(19) Europäisches Patentamt / European Patent Office / Office européen des brevets

(11) **EP 2 143 775 A1**

(12) **EUROPEAN PATENT APPLICATION**


(43) Date of publication:
**13.01.2010 Bulletin 2010/02**

(21) Application number: **08008782.8**

(22) Date of filing: **09.05.2008**

(51) Int Cl.:
***C09K 11/06*** *(2006.01)* ***H05B 33/14*** *(2006.01)*
***C07C 49/755*** *(2006.01)* ***C08G 61/10*** *(2006.01)*
***H01L 51/00*** *(2006.01)*

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**



(71) Applicants:
- **Max-Planck-Gesellschaft zur Förderung der Wissenschaften e.V.**
  **80539 München (DE)**
- **Technische Universität Graz**
  **8010 Graz (AT)**

(72) Inventors:
- **Müllen, Klaus**
  **50939 Köln (DE)**
- **Qin, Tianshi**
  **55122 Mainz (DE)**
- **Bauer, Roland**
  **85386 Eching (DE)**
- **List, Emil J.W.**
  **8010 Graz (AT)**
- **Scheiber, Horst**
  **9900 Lienz (AT)**

(74) Representative: **Katzameyer, Michael**
**v. Bezold & Partner**
**Patentanwälte**
**Akademiestrasse 7**
**80799 München (DE)**


(54) **Blue light emmiting polythriphenylene dendrimers, methods of preparation and uses thereof**


(57) The present invention relates to novel blue light emitting polytriphenylene dendrimers, methods of preparation and uses thereof.

The present polytriphenylene dendrimers comprise a core unit with linked diphenyltriphenylene repeating units of the following formula (1)

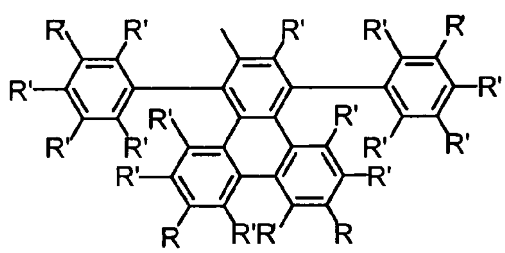

The present invention further provides a method for preparing such dendrimers via a series of Diels-Alder cycloadditions reacting an alkinyl-functionalized compound with a diphenylcyclopentaphenanthrenone of formula (2)


EP 2 143 775 A1

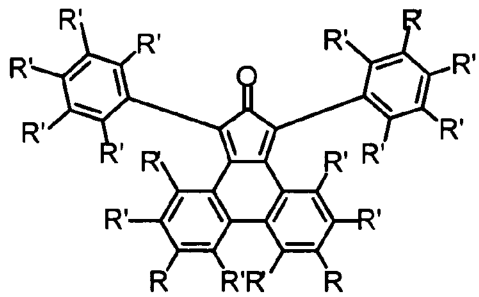

(2)

Also provided is a novel method for preparing the diphenylcyclopentaphenanthrenone of formula (2).

## Description

In the field of organic light emitting polymers it is widely recognized that the band gap and thus the absorption and emission wavelengths of conjugated polymers of a general formula largely depend upon the nature of the repeat units and of the coupling units.

The most common examples involve benzene as building blocks which are coupled directly via ethylene or ethynylene moieties. The occurrence of extended $\Pi$-conjugation can be compromised by strong torsion about formal single bonds due to sterical hindrance under the influence of bulky alkyl substituents. A good case can be made by considering poly(para-phenylene)s due to their wide band gaps which are important blue light emitters. Here, solubility and solution processability are brought about by alkyl substituents, which, however, inhibit extended $\Pi$-conjugation. This is why stepladder polymers, such as polyfluorenes and ladder polymers, such as poly(ladder-type tetraphenylene) s have played an important role in the search for blue light emitters.

Blue emission can also be obtained by using low molecular weight chromophores based upon polycyclic aromatic hydrocarbons (PAH's) such as anthracene, phenanthrene or pyrene. A major obstacle for their use in light emitting diodes is the formation of crystallites in the solid and thus the occurrence of light scattering. Amorphous films of such materials can be obtained by the introduction of bulky alkyl or aryl substituents which inhibit tight intermolecular packing of the chromophores.

PAH's can also be incorporated into conjugated polymers whereby the critical issue is, again, the degree of their conjugative interaction along the chain. Similar to the conjugated polymers the attachment of bulky substituents improves solubility and ensures the amorphous character of films, but also "dilutes" the optical function of the material.

Among the known PHA chromophores with blue light emitting capacity are triphenylene derivatives which are have been extensively studied for a few decades with regard to their discotic liquid crystallinity and their potential function as electronic materials in light emitting diodes (Freudenmann et al., J. Mat. Chem. 2001, 11, 1618; WO 2006/130598), field-effect transistors, and photovoltaic cells. However, the high tendency towards self-association of triphenylenes often leads to a dramatic decrease in fluorescence intensity as consequence.

Triphenylene units have also been used as the core unit in dendrimer structures (WO 2008/012250). Monodisperse dendrimers represent a fast-growing field of research in macromolecular organic chemistry. In the past decades, the design and synthesis of $\pi$-conjugated dendrimers have been explored extensively due to their unique molecular structures. In comparison with linear $\pi$-conjugated oligomers and polymers, rigid dendritic architectures furnish dendrimers with new morphological, electrical and photophysical properties (Bauer et al., Top. Curr. Chem. 2005, 245, 253). The dendrimers in the art comprise a broad range of possible structures for different applications but lack blue light emitting chromophores.

In view of the drawbacks of the blue light emitters of the prior art there is clearly a need for novel blue light emitting polymers with amorphous character and improved emission properties and the main object of the present invention is to provide such polymers.

The present inventors have adressed this need by synthesizing a new class of polytriphenylene dendrimers which are based prevalently or, preferably, exclusively on interlinked triphenylene units and have been found to exhibit excellent thermal stability, favourable pure-blue emission spectra and high photoluminescence quantum yields (PLQY).

According to the best knowledge of the present inventors, such polytriphenylene dendrimers have never been realized before, probably due to the synthetic challenge associated with the preparation of functionalized triphenylene derivatives suitable for the employment as branching reagents in dendrimer and hyperbranched polymer synthesis.

Thus, the above main object of the invention has been achieved by providing the polytriphenylene dendrimers of claims 1-6 and the method of preparing the same according to claims 7-12. Related objects have been achieved by providing a novel method for peparing the diphenylcyclopentaphenanthrenone starting compound according to claim 13, the emissive layer according to claim 14, the uses according to claim 15 and the opto-electronic devices according to claim 16.

In the present dendrimers the triphenylene units are twisted against each other leading to an inherent stiffness of the macromolecules. By importing the triphenylene units into a stiff dendritic matrix any disadvantageous aggregation of triphenylene units can be avoided. Such polyphenylene dendrimers with highly stiff and shape-persistent dendritic backbones also suppress inter-chromophore interactions.

Summarizing, the novel polytriphenylene dendrimers of the present invention exhibit a number of favourable characteristics:

(i) blue light emission is brought about by the presence of electronically decoupled PAH units;
(ii) which are incorporated into a rigid polyphenylene dendrimer and thus adopt sterically defined positions; disallowing intra-dendrimer chromophore-chromophore interactions;
(iii) amorphous films are obtained due to the lack of intermolecular interactions, and
(iv) the amount of "useless" substituents and coupling units can be kept at a minimum.

Furthermore, the synthetic approach for preparing the present polyphenylene dendrimers as used therein allows the accurate control over the number of chromophores within one molecule. The synthesis does not require any catalyst and can be effected in quantitative or nearly quantitative yield.

The novel polytriphenylene dendrimers as defined in claim 1 comprise a core unit with linked diphenyltriphenylene repeating units of the following formula (1)

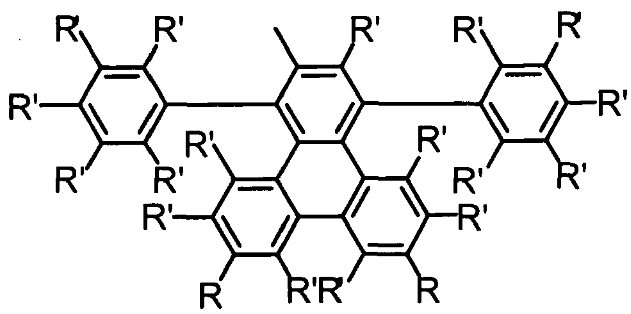

wherein each R' independently is H or a substituent which does not participate in a Diels-Alder reaction, each R independently is H, a substituent which does not participate in a Diels-Alder reaction, an ethynyl group or protected ethynyl group, or a diphenyltriphenylene unit of said formula (1).

The above substituents R or R' which do not participate in a Diels-Alder reaction may be selected from the group consisting of aryl, substituted aryl, heteroaryl, substituted heteroaryl, alkyl, alkylaryl, amino, halo, OH, carboxy, alkoxy, ester. More preferably, the substituents R or R' which do not participate in a Diels-Alder reaction are selected from the group consisting of phenyl, substituted phenyl, alkyl, such as alkyl with 1-10 carbon atoms, more preferably 1-6 carbon atoms, amino, halo, OH, carboxy, alkoxy, ester groups.

In a preferred specific embodiment of the present invention, the polytriphenylene dendrimer comprises one of the following structures:

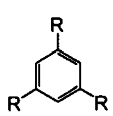 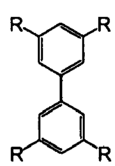 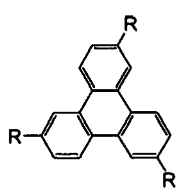 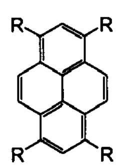 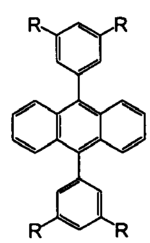 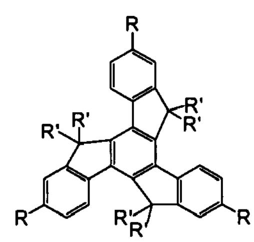

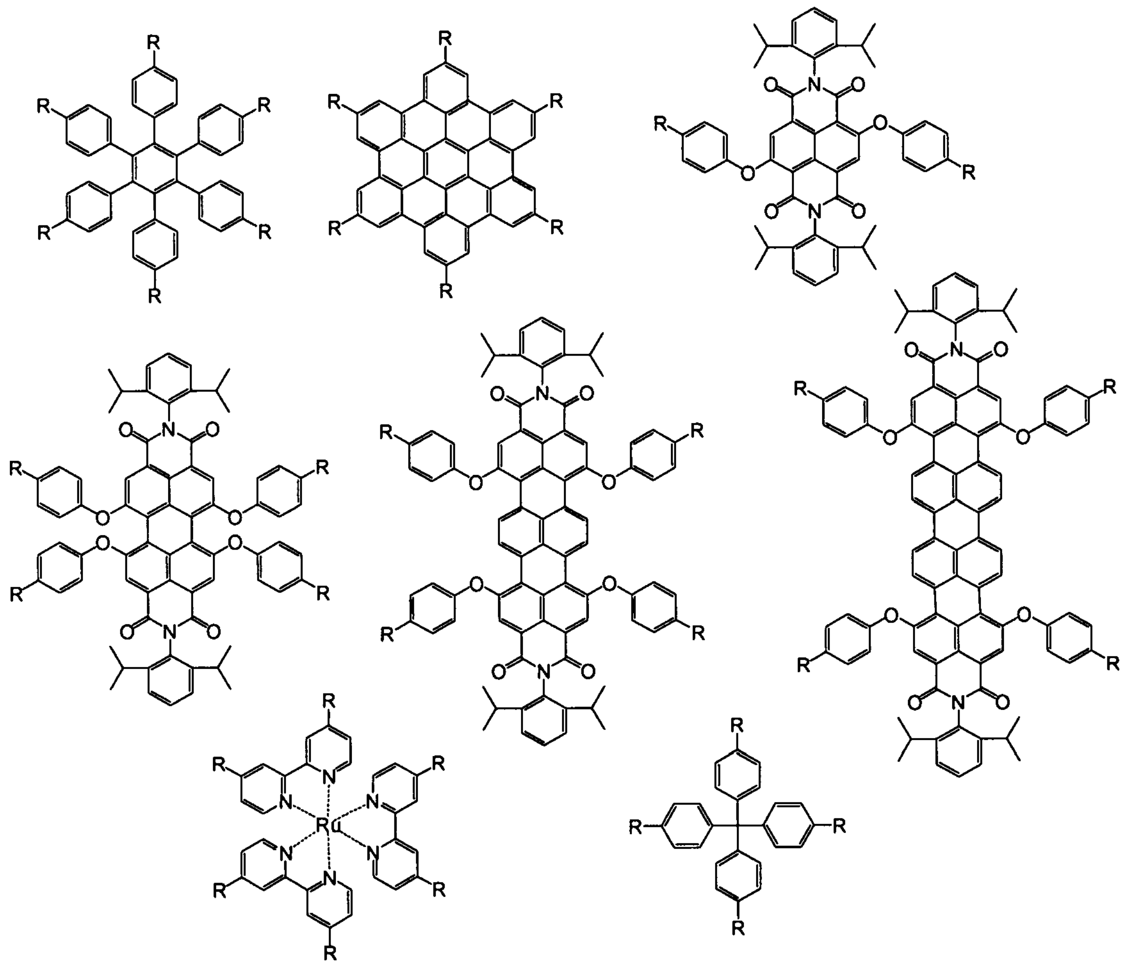

wherein each substituent R of the core unit independently is a polytriphenylene dendron consisting of one or more diphenyltriphenylene units of formula (1) as defined above and each R', if present, independently is H or a substituent which does not participate in a Diels-Alder reaction.

In a more specific embodiment of the present invention, the polytriphenylene dendrimer comprises a compound of the following formulae (G01) or (G02)

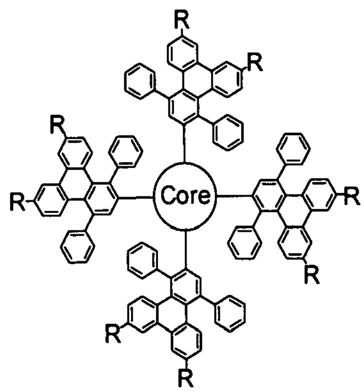

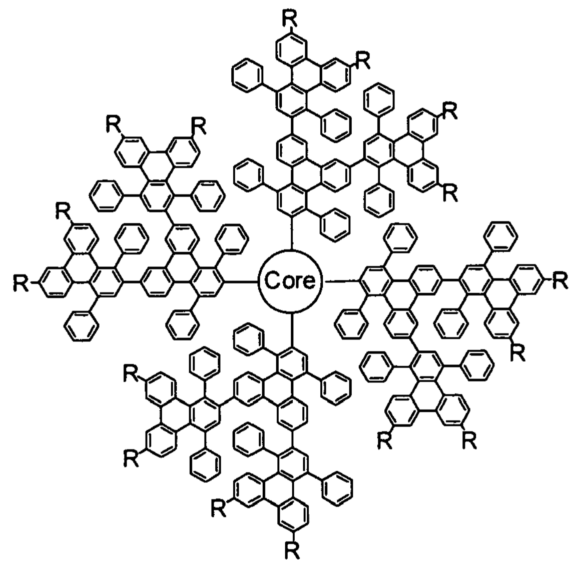

wherein all substituents R are the same and selected from H, a substituent which does not participate in a Diels-Alder reaction as defined above, an ethynyl group or protected ethynyl group or a diphenyltriphenylene unit of the formula (1) as defined above.

Still more specifically, the polytriphenylene dendrimer is represented by one of the following formulae

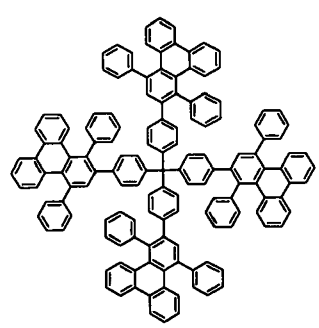

G1

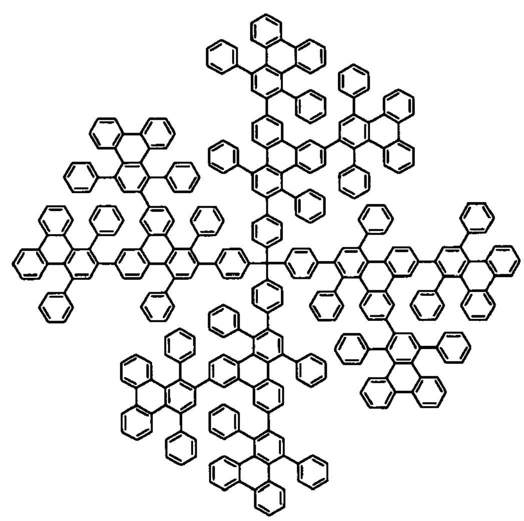

G2

or

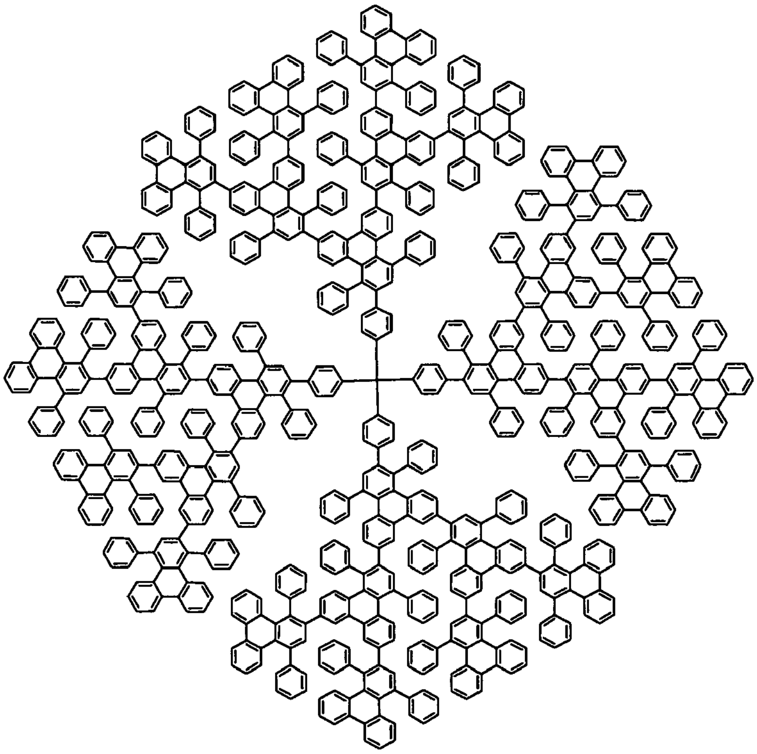

G3 .

Advantageously, the polytriphenylene dendrimers acccording to the present invention will be capable of blue light electroluminescence emission. Typically, the maximum im the emission spectra of the polytriphenylene dendrimers will be in a range from 380-470 nm, preferably from 400-440 nm. The photoluminescense quantum yields (PLQY) may be in a range from 3 to 80 %, more specifically from 5 to 50 %.

The key strategy in the synthesis of the present polytriphenylene dendrimers with separate triphenylene chromophores is the use of a diphenylcyclopentaphenanthrenone derivative which represents an $AB_2$-type branching compound combining a diene function and two dienophil ethynyl functions in one and the same molecule. The reaction of such a diphenylcyclopentaphenanthrenone with a compound having at least one reactive alkinyl group in a Diels-Alder [4+2] cycloaddition results in a diphenyltriphenylene structure representing the basic repeating unit in the present dendrimer synthesis.

The ethynyl functions, when protected, e.g. substituted with triisopropylsilyl (TiPS) groups, cannot enter Diels-Alder cycloaddition so that the diphenylcyclopentaphenanthrenone can be added to any kind of multi-ethynyl core to double the amount of ethynyl functions by a sequence of Diels-Alder cycloaddition. After removal of the TiPS-protecting groups, the divergent growth of the dendrimer can be repeated upon of addition of, again, the $AB_2$-system.

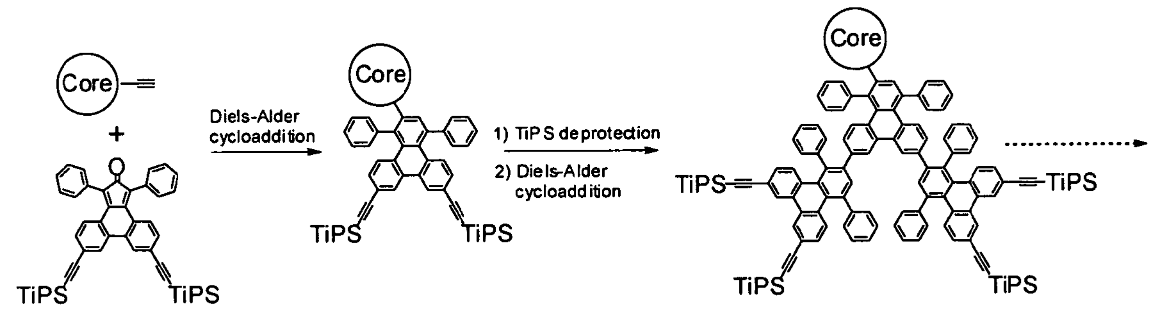


**Scheme 1**

A novel and especially advantageous method for synthesizing the diphenylcyclopentaphenanthrenone of formula (2)

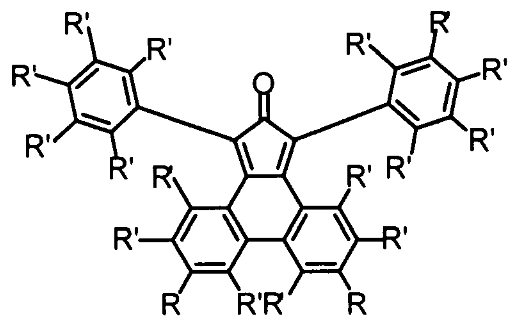

(2)

wherein each R' independently is H or a substituent which does not participate in a Diels-Alder reaction, and each R independently is H, a substituent which does not participate in a Diels-Alder reaction, or a protected ethynyl group, comprises the following steps:

a) brominating an unsubstituted or substituted phenanthrene-9,10-dione to give the corresponding 3,6-dibromophenanthrene-9,10-dione;
b) protecting the dione group with a protecting agent, e.g. dimethylsulfate, to give a protected derivative, e.g. 3,6-dibromo-9,10-dimethoxyphenanthrene;
c) reacting the product of step b), e.g. 3,6-dibromo-9,10-dimethoxyphenanthrene, with a compound comprising a protected ethynyl group, e.g. ethynyltriisopropylsilane or ethynyltrimethylsilane, to give the corresponding ethynyl-functionalized product, e.g. 9,10-dimethoxy-3,6-bis(triisopropylsilyl)ethynyl)phenanthrene or 9,10-dimethoxy-3,6-bis(trimethyl)ethynyl)phenanthrene;
d) removing the methoxy groups (or other protecting groups of step b)) by reacting with an oxidating agent to give a 3,6-bis(triisopropylsilyl)ethynyl)phenanthrene-9,10-dione or an analogous compound with another ethynyl-protecting group;
e) reacting the product of step d) with 1,3-diphenylacetone under Knoevenagel condensation to give the diphenylcyclopentaphenanthrenone of the above formula (2) wherein each R is a protected ethynyl group, e.g. a triisopropylsilylethynyl group, and
f) optionally removing the protective groups, e.g. triisopropylsilyl groups, to give the diphenylcyclopentaphenanthrenone of the above formula (2) wherein each R is an ethynyl group.

The present method for synthesisizing a polytriphenylene dendrimer of the present invention generally comprises:
reacting a compound comprising at least 2, 3 or 4 reactive ethynylphenyl groups and a 1,3-diphenylcyclopentaphenanthrenone of the formula (2)

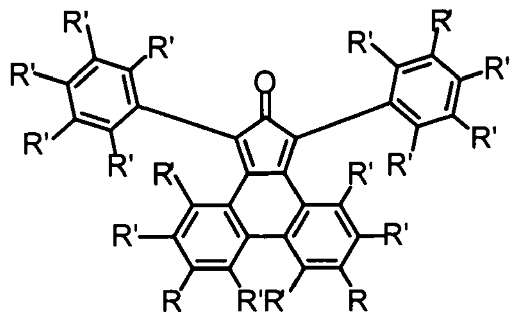

(2)

wherein each R' independently is H or a substituent which does not participate in a Diels-Alder reaction, and
each R independently is H, a substituent which does not participate in a Diels-Alder reaction, or a protected ethynyl group, in a Diels-Alder cycloaddition to form a polytriphenylene dendrimer.

More specifically, in this method both R in formula (2) are the same and the resulting polytriphenylene dendrimer has one of the exemplary structures shown above on page 6.

For preparing polytriphenylene dendrimers of a higher order, in the above reaction both R in formula (2) are a protected ethynyl group and the Diels-Alder cycloaddition results in a first generation polytriphenylene dendrimer comprising protected ethynyl groups R, and the method further comprises

a) deprotecting the ethynyl groups and
b) reacting the deprotected ethynyl groups with the 1,3-diphenylcyclopentaphenanthrenone of the formula (2) as defined above or with a tetraphenylcyclopentadienon in a Diels-Alder cycloaddition to form a second generation polytriphenylene dendrimer and,
c) if the second generation polytriphenylene dendrimer comprises protected ethynyl groups R due to the presence of such groups in the diphenylcyclopentaphenanthrenone of the above formula (2) (or in the tetraphenylcyclopentadienon) used in step b), optionally repeating steps a) and b) once or several times to form polytriphenylene dendrimers of a third or higher generation.

Preferably, in said method the substituents R of the diphenylcyclopentaphenanthrenone of formula (2) used in step b) and/or step c) are the same.

In a more specific embodiment of the present invention, the method comprises:
reacting tetrakis(4-ethynylphenyl)methane and a 1,3-diphenylcyclopentaphenanthrenone of the formula (201)

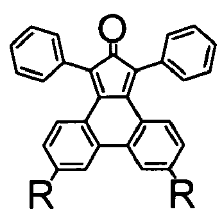

wherein all substituents R are the same and selected from H, an ethynyl group or protected ethynyl group,
in a Diels-Alder cycloaddition to form a polytriphenylene dendrimer of the following formula (G01)

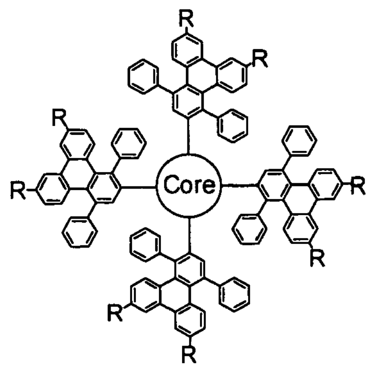

wherein all substituents R are the same and selected from H, an ethynyl group or protected ethynyl group.

For preparing polytriphenylene dendrimers of a higher order, said method further comprises

a) reacting the polytriphenylene dendrimer of formula (G01)

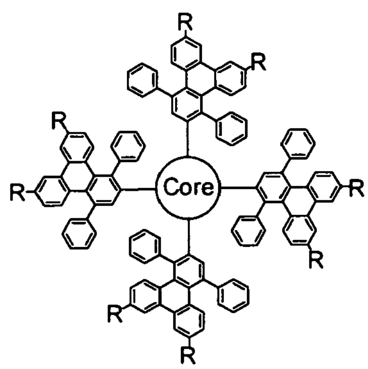

wherein each R is a protected ethynyl group,
with a deprotecting agent to form a polytriphenylene dendrimer of formula (G01)

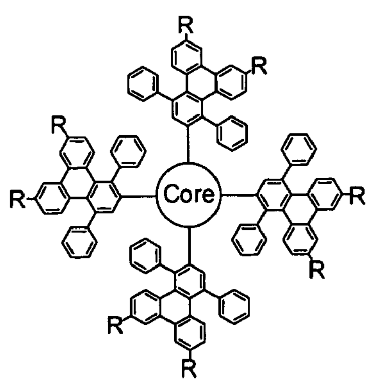

wherein R is an ethynyl group, and

b) further reacting the dendrimer obtained in step a) with a tetraphenylcyclopentadienon or, preferably, with a 1,3-diphenylcyclopentaphenanthrenone of the formula (201)

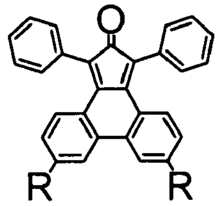

wherein R may be H or a protected ethynyl group to form a second generation polytriphenylene dendrimer, and

c) if the second generation polytriphenylene dendrimer comprises protected ethynyl groups R due to the presence of such groups in the diphenylcyclopentaphenanthrenone of the above formula (201) (or in the tetraphenylcyclopentadienon), optionally repeating steps a) and b) once or several times to form polytriphenylene dendrimers of a third or higher generation.

Specifically, for the synthesis of the first-generation dendrimer **G1** (Scheme 3) with four chromophores, the phencyclone **7** of Scheme 3 was reacted with tetrakis(4-ethynylphenyl)methane in o-xylene at 170 ˚C in microwave reactor for 2-4 hours. After the removal of the excess of **7** by repetitive precipitation in methanol, the first-generation dendrimer **G1** was recrystallized from a mixture of tetrachloroethane and hexane. The synthesis of the higher generation polytriphenylene dendrimers (Scheme 3) was carried out similarly following the corresponding synthetic protocol. Accordingly by the use of the $AB_2$ branching agent **6** in iterative Diels-Alder/deprotection sequences dendrimers **G2** with twelve triphenylene and **G3** with twenty-eight triphenylene units could be synthesized in good yields.

Summarizing, the present invention provides a novel series of efficient pure blue emitting dendrimers. The synthesis from $AB_2$ type building blocks to polytriphenylene dendrimers is non-catalyst and in quantitive yield. Most importantly, the twisted triphenylene units not only function as chromophores, but also effectively prevent the inter- or intramolecular fluorescence quenching, as proved by increased PLQY with increasing generation. These dendrimers exhibit stable and pure-blue emission in both PL and EL spectra and they could provide an avenue for dendritic emitters with the optimized EL efficiency/color purity trade-offs needed for pure blue light, which is still a great challenge in the field of blue-emitting materials.

The present polytriphenylene dendrimers have applications in all technical fields where blue light emitters with the above favorable charactistics are needed. Such fields include generally opto-electronic devices and in particular organic LED devices, organic photodetectors, organic phototransistors, FET and solar cells.

In a related aspect, therefore, the present invention provides also opto-electronic devices, and in particular LED devices, organic photodetectors, organic phototransistors, FET and photovoltaic cells, comprising the present polytriphenylene dendrimers. In a further related aspect of the present invention, an emissive layer comprising the present polytriphenylene dendrimers is provided.

In opto-electronic devices as exemplified above, the dendrimers according to the present invention are used as electroluminescent material. The dendrimers may be present in the device, in particular the emissive layer, as the sole ligt emitting polymer or as a component in a blend further comprising hole and/or electron transporting polymers.

In a preferred embodiment, the device comprises distinct layers of a dendrimer of the present invention, a hole transporting polymer and/or an electron transporting polymer. These layers will be located on a substrate between a charge injecting layer for injecting positive charged carriers, such as an anode electrode layer, and a charge injecting layer for injecting negative charge carriers, such as a cathode electrode layer. Additionally, charge transport layers may be provided between the emissive layer and one or both of the charge injecting layers.

In one preferred embodiment, the opto-electronic device comprises an assembly of at least

a) Hole injecting electrode (anode)
b) Hole transporting layer
c) Light emitting dendrimer of the invention
d) Electron injecting electrode (cathode) on a transparent substrate.

An example of such an assembly is depicted in Fig. 5.

In another preferred embodiment, additionally at least one hole blocking/ electron transporting layer is provided between c) and d).

The anode may be of any material used in the art for this purpose. Suitable examples are metals, such as gold, silver, indium, iron, zinc, tin, metallic alloys such as magnesium/copper, aluminium/indium etc., semiconductors such as Si, Ge, etc., metallic oxides such as indium-tin-oxide or electroconducting polymers such as polyaniline etc. (for

further examples see, e.g. WO 2008/012250) . In a typical embodiment, the anode material is a mixed oxide of tin and indium (ITO) which is deposited on a transparent substrate such as glass, quartz or plastics, such as PET, so that the light emitted by the organic film comprising the emissive layer my be observed. The organic film may be the composite of several individual layers as outlined above, each designed for a distinct function. Since holes are injected from the anode, the layer next to the anode needs to have the functionality of transporting holes. Similarly, the layer next to the cathode needs to have the function of transporting electrons. The emissive layer comprising the present dendrimers can perform the combinded functions of electron transport and light emission. Still, an additional electron transporting layer may be present.

The hole transporting layer may comprise any polymer known in the art for this purpose (e.g. as disclosed in WO 2008/012250). In a specific embodiment of the invention, the hole transporting layer comprises poly(3,4-ethylenedioxy thiophene) (PEDOT) and poly(styrene sulfonic acid) (PSS).

The optional electron transporting layer may comprise any low molecular weight materials or polymers known in the art for this purpose (e.g. as disclosed in WO 2008/012250). Suitable compounds may be, e.g., 1,3,4-oxadiazoles or 1,3, 4-oxadiazole-containing polymers. In a specific embodiment of the invention, a layer of 4,4',4''-tris(N-3-methylphenyl-N-phenyl-amino)triphenylamine TPBi) is used.

The cathode material be of any material used in the art for this purpose (e.g. as disclosed in WO 2008/012250). Some non-limiting examples are silver, aluminium, alkali metals, earth alkali metals, and alloys thereof, and electroconducting polymers such as polyprrrole, polythiophene etc. In a specific embodiment, the cathode is made of a Ca/Al alloy or CsF/Al.

The different layers may be deposited by any suitable method known in the art. For example, the PEDOT/PSS films and emissive dendrimer films may be spin-cast on the substrate and layers of metals or metallic alloys may be deposited by vapor deposition. Other suitable methods are e.g. disclosed in WO 2008/012250.

The invention is further illustrated by the following non-limiting Examples and Figures.

FIGURES

**Fig. 1.** MALDI-Mass spectra for dendrimers **G1**, **G2** and **G3.**

**Fig. 2.** Crystallographic structure of dendrimer **G1**.

**Fig. 3.** UV-vis absorption and PL spectra ($\lambda_{ex}$ = 300 nm) of dendrimers in chloroform solutions (a) and in solid states (b).

**Fig. 4.** *I-V-L* characteristics of an light emitting ITO/PEDOT:PSS/**G2**/TPBi(10nm)/CsF/Al device. Inset: normalized electroluminescence spectrum at 8V bias.

**Fig. 5.** Assembly of an exemplary OLED device:

1) transparent substrate (e.g. glass)
2) Hole injecting electrode (e.g. ITO)
3) Hole transporting layer (e.g. PEDOT/PSS)
4) Light emitting dendrimer of the invention (e.g G2, G3)
5) Electron injecting electrode (e.g. Ca/Al or CsF/Al)

EXAMPLE 1

Synthesis of 1,3-diphenyl-6,9-bis((triisopropylsilyl)ethynyl)-cyclophenanthrenone (6)

The synthesis of the intermediate compounds 2, 3, 4 and 5 and diphenylcyclopentaphenanthrenone **6** is shown in scheme 2 below.

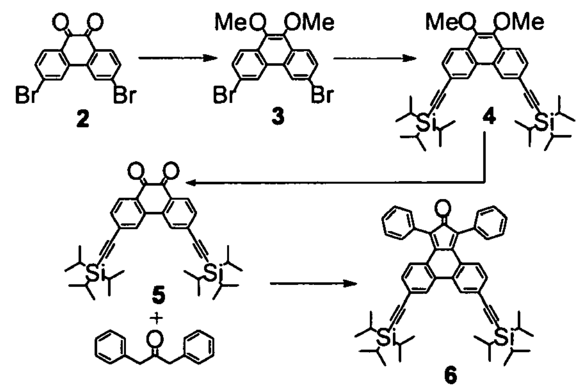
2 3 4 5 6

**Scheme 2**

The synthesis started with the commercially available phenanthrene-9,10-dione which was brominated to give 3,6-dibromophenanthrene-9,10-dione **2** in bromine. Due to the instability of the dione group during Hagihara-Sonogashira coupling, it was necessary to protect the dione group of compound **2** with dimethyl sulfate in order to provide 3,6-dibromo-9,10-dimethoxyphenanthrene **3** which subsquently was treated with ethynyltriisopropylsilane under Hagihara-Sonogashira conditions to afford 9,10-dimethoxy-3,6-bis((triisopropylsilyl)-ethynyl)phenanthrene **4.** The dimethoxy group was cleaved via an efficient oxidation by using cerium ammonium nitrate (CAN) to yield 3,6-bis((triisopropylsilyl)ethynyl) phenanthrene-9,10-dione **5.**

The final step under Knoevenagel condensation of **5** with 1,3-diphenylacetone gave the key intermediate building block **6.**

**3,6-dibromo-9,10-dimethoxyphenanthrene (3):** Compound **3** was prepared using a published procedure for related compounds. Compound **2** (4.25 g, 11.6 mmol), $Bu_4NBr$ (1.48 g, 4.6 mmol), $Na_2S_2O_4$ (8.20 g, 47.0 mmol), THF (100 mL) and $H_2O$ (100 mL) were combined in a 500 mL round bottom flask and shaken for 5 min, after which dimethyl sulfate (7.5 mL, 79.5 mmol) was added, followed by aqueous sodium hydroxide (20 mL, 14.0 M). The mixture was stirred for 3 min, during which, 50 g of ice was added, then the mixture was additionally stirred for 15 minutes. The aqueous layer was separated and extracted with ethyl acetate (EtOAc) (3 × 750 mL) . The combined organic layers were washed with water (3 x 100 mL), $NH_4OH$ solution (2 × 500 mL) and brine (1 × 500 mL). The organic layer was dried with $MgSO_4$, filtered and the solvents were removed under vacuum, resulting in a fluffy yellow solid. Washing the product with methanol (MeOH) gave a white solid. Additional impurities were removed by flashing the product through silica with a 1:1 mixture of hexanes and dichloromethane (DCM). Yield: 4.27 g, 10.8 mmol, 92%.

$^1$H NMR (250 MHz, $CD_2Cl_2$) δ 8.69 (d, 2H, *J* = 1.8 Hz, aromatic C*H*), 8.10 (d, 2H, *J* = 8.8 Hz, aromatic C*H*), 7.72 (dd, 2H, *J*1 = 8.8 Hz, *J*2 = 1.8 Hz, aromatic C*H*), 4.06 (s, 6H, OC*H*$_3$); $^{13}$C NMR (62.5 MHz, $CD_2Cl_2$) δ 144.2, 130.9, 129.2, 128.8, 125.8, 124.5, 120.7, 61.3; FD-MS: *m*/*z* = 398.1 ($[M]^+$).

**9,10-dimethoxy-3,6-bis((triisopropylsilyl)ethynyl)phenanthrene (4):** Compound **3** (3.4 g, 8.6 mmol), Pd $(PPh_3)_2Cl_2$ (0.68 g, 0.96 mmol), CuI (0.38 g, 2.0 mmol) and $PPh_3$ (0.52 g, 2.0 mmol) were dissolved in 100 mL of toluene/triethylamine solution and degassed by three freeze/pump/thaw cycles. The solution was heated to reflux under argon, 5 mL (33.8 mmol) triisopropylsilylacetylene was added by syringe quickly to obtain a brown solution. The solution was kept stirring at 80 °C for 16 h. The solution was filtered and the solvent was removed by vacuum. After redissolution in DCM, the solution was washed with $NH_4Cl$, 1M HCl, and $NaHCO_3$. The organic phase was evaporated and purified by column chromatography (silica gel, PE: DCM = 4:1). Yield: 4.32 g, 7.22 mmol, 84%.

$^1$H NMR (250 MHz, $CD_2Cl_2$) δ 8.73 (s, 2H, aromatic C*H*), 8.14 (s, 2H, *J* = 8.5 Hz, aromatic C*H*), 7.68 (d, 2H, *J*1 = 8.4 Hz, aromatic C*H*), 4.07 (s, 6H, OC*H*$_3$), 1.19 (s, 42H, TiPS *CH$_2$ and CH$_3$*); $^{13}$C NMR (62.5 MHz, $CD_2Cl_2$) δ 144.9, 130.7, 129.5, 128.0, 126.8, 122.6, 121.4, 107.8, 92.0, 61.3, 18.9, 11.8; MALDI-TOF: *m*/*z* = 598.5 ($[M]^+$).

**3,6-bis((triisopropylsilyl)ethynyl)phenanthrene-9,10-dione (5):** Compound **4** (2.80 g, 4.6 mmol) was dissolved in acetonitrile (20 mL) and DCM (20 mL) under argon protection, then the mixture was added with stirring ammonium cerium (IV) nitrate (6.20 g, 11. 4 mmol) in water (60 mL) over 15 min. The reaction was diluted with 100 mL H2O and extracted with 3 × 100 mL DCM, and the organic phase was concentrated and purified by column chromatography (silica gel, PE: DCM = 1:1), affording an orange powder. Yield: 2.24 g, 3. 94 mmol, 84%.

$^1$H NMR (250 MHz, $CD_2Cl_2$) δ 8.12 (d, 2H, *J*1 = 1.9 Hz, aromatic C*H*), 8.10(s, 2H, aromatic C*H*), 7.56 (d, 2H, *J*1 = 8.1 Hz, aromatic C*H*), 1.18 (s, 36H, silyl *CH$_3$),* 1.17 (s, 6H, *CH$_2$*); $^{13}$C NMR (62.5 MHz, $CD_2Cl_2$) δ 197.9, 144.9, 130.7, 129.5, 128.0, 126.8, 122.6, 121.4, 107.8, 92.0, 18.9, 11.8; FD-MS: *m*/*z* = 568.8 ($[M]^+$).

**1,3-diphenyl-6,9-bis((triisopropylsilyl)ethynyl)-cyclopenta-phenanthrenone (6):** Compound **5** (1.36 g,

2.4 mmol) and 1,3-diphenyl-2-propanone (0.75 g, 3.6 mmol) were dissolved in anhydrate MeOH (50 mL) under argon atmosphere, 0.14 g KOH methanolic solution was added dropwise, and the mixture was heated up to 80 ˚C for 5 min. The brown color mixture was fast frozen to 0 ˚C and 1.9 mL HCl (1.25 M in MeOH) was added with stirring to neutralize the pH to 5. The green precipitated was filtered, washed with cold MeOH and purified by colunm chromatography (silica gel, PE:DCM = 2:1), affording a dark green powder. Yield: 1.08 g, 1.45 mmol, 61%. $^1$H NMR (250 MHz, $CD_2Cl_2$) δ 7.92 (s, 2H, aromatic *CH*), 7.48 (s, 2H, aromatic *CH*), 7.43 (dd, 4H, *J*1 = 9.7 Hz, *J*2 = 2.2 Hz, aromatic C*H*), 7.40 (s, 2H, aromatic *CH*), 7.34 (dd, 4H, *J*1 = 9.4 Hz, *J*2 = 1.6 Hz, aromatic C*H*), 7.03 (dd, 2H, *J*1 = 9.6 Hz, *J*2 = 1. 3 Hz, aromatic C*H*), 1.14 (s, 42H, silyl $CH_3$ and $CH_2$); $^{13}$C NMR (62.5 MHz, $CD_2Cl_2$) δ 197.8, 147.4, 137.8, 136.2, 134.4, 133.0, 132.6, 132.1, 130.2, 129.0, 128.7, 128.6, 128.2, 127.1, 126.7, 124.2, 123.8, 106.8, 94.9, 93.9, 18.8, 11.7; FD-MS: *m*/*z* = 743.7 ($[M]^+$).

EXAMPLE 2

Synthesis of 1st generation dendrimers

The synthesis of the 1st generation dendrimers G1, 8 and 9 is shown in Scheme 3.

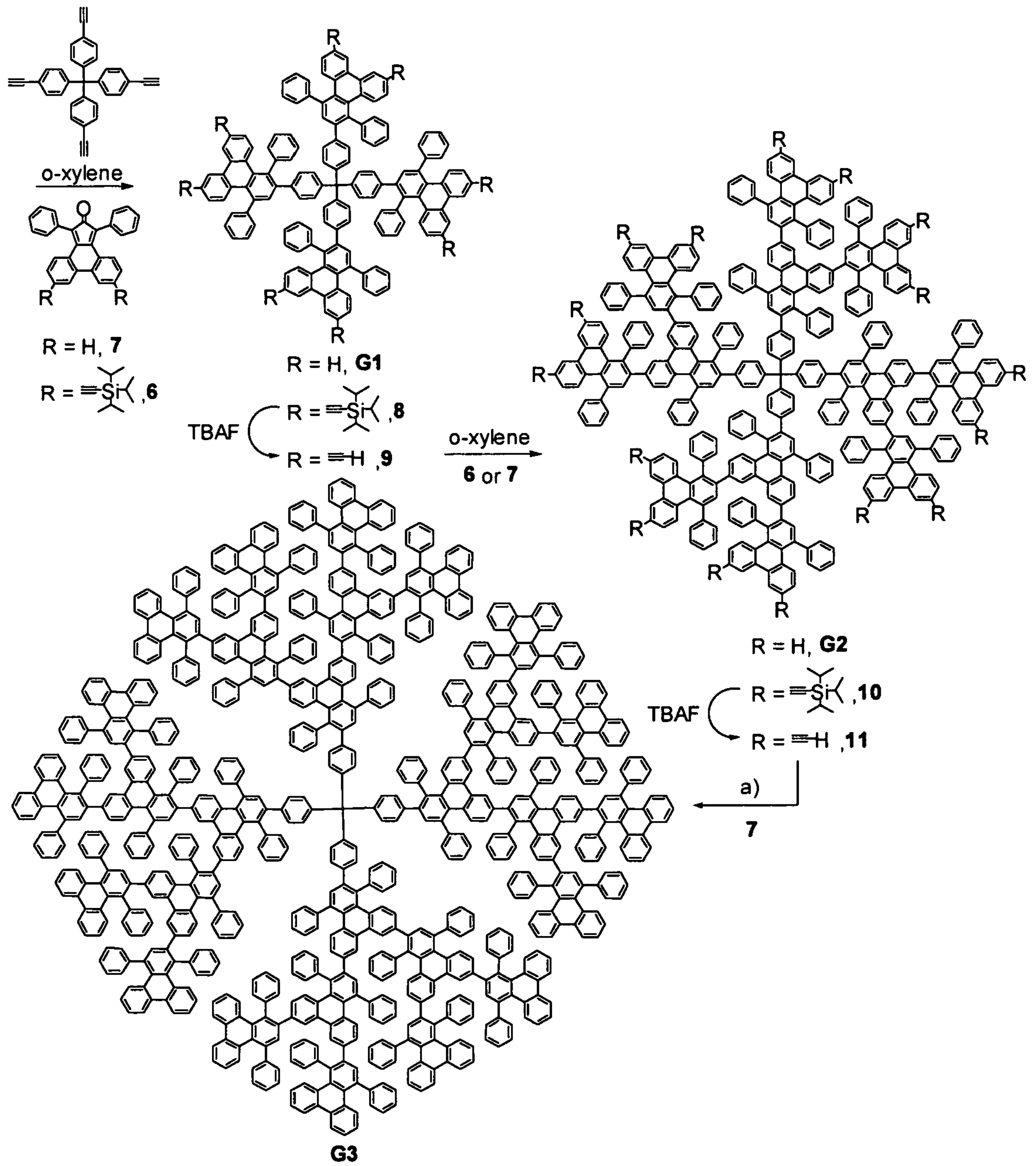


**Scheme 3**

## $1^{st}$ generation polytriphenylene dendrimer (G1):

Tetrakis(4-ethynylphenyl)methane (0.060g, 0.144 mmol) and **7** (0.440 g, 1.152 mmol,) were put under Argon, dissolved in o-xylene (2 mL) in a microwave tube and stirred at 170 ˚C for 4 h. After cooling to RT, the reaction mixture was precipitated in MeOH, then washed with DCM till the green color disappeared, affording a white powder. Yield: 0.255 g, 0.140 mmol, 97%.

$^1$H NMR (700 MHz, $C_2D_2Cl_4$) δ 8.35 (t, 8H, , *J* = 7.1 Hz, aromatic *H*1), 7.69 (s, 4H, aromatic *H*2), 7.64 (d, 4H, *J* = 8.5 Hz, aromatic *H*3), 7.50 (t, 12H, , *J* = 8.8 Hz, aromatic *H*4), 7.40 - 7.32 (n.r., 20H), 7.12 (d, 4H, *J* = 7.3 Hz, aromatic *H*5), 7.08 (t, 4H, *J* = 14.8 Hz, aromatic *H*6), 7.04 (d, 12H, *J* = 7.4 Hz, aromatic *H*7), 6.96 (t, 4H, *J* = 7.7 Hz, aromatic *H*8), 6.89 (d, 8H, *J* = 8.2 Hz, aromatic *H*9), 6.81 (d, 8H, *J* = 8.2 Hz, aromatic *H*10); $^{13}$C NMR (125 MHz, $C_2D_2Cl_4$) δ 145.0, 144.8, 144.7, 142.5, 142.4, 142.4, 140.6, 140.3, 140.1, 140.0, 139.9, 138.6, 137.4, 137.0, 132.8, 132.5, 132.4, 132.2, 132.0, 131.92, 131.7, 131.5, 131.1, 131.1, 130.8, 130.5, 130.4, 130.2, 130.0, 129.5, 129.4, 129.3, 129.2, 129.1, 128.8,

128.7, 128.7, 127.7, 127.6, 127.5, 127.4, 127.3, 127.0, 126.9, 126.7, 125.9, 125.5, 124.8, 123.5; MALDI-TOF ($Ag^+$): *m/z* = 1941.1 ($[M+Ag]^+$).

**1st generation T*i*PSethynyl polytriphenylene dendrimer (8):**

Tetrakis (4-ethynylphenyl) methane (0.042 g, 0.100 mmol) and **6** (0.350 g, 0.472 mmol,) were put under Argon, dissolved in o-xylene (2 mL) in a microwave tube and stirred at 170 ˚C for 4 h. After cooling to RT, the reaction mixture was evaporated *in vacuo.* The residue was further purified by column chromatography (silica gel, PE:DCM = 6:1), affording light yellow powder. Yield: 0.266 g, 0.081 mmol, 81%.

$^1$H NMR (500 MHz, $CD_2Cl_2$) δ 8.55 (d, 8H, , *J* = 2.3 Hz, aromatic *H*1), 7.78 (s, 4H, aromatic *H*2), 7.64 (d, 4H, *J* = 8.7 Hz, aromatic *H*3), 7.52 (t, 12H, , *J* = 8.4 Hz, aromatic *H*4), 7.43 (dd, 12H, *J*1 = 3.2 Hz, *J*2 = 10.6 Hz, aromatic *H*5), 7.21 - 7.15 (n.r., 16H), 7.08 (d, 12H, *J* = 6.9 Hz, aromatic *H*6), 6.98 (d, 8H, *J* = 8.5 Hz, aromatic *H*7), 6.87 (d, 8H, *J* = 8.5 Hz, aromatic *H*8), 1.14 (s, 168H; TiPS); $^{13}$C NMR (125 MHz, $CD_2Cl_2$) δ 149.0, 148.7, 146.9, 146.4, 145.1, 145.0, 144.4, 144.3, 144.1, 143.4, 143.0, 141.8, 141.7, 137.7, 136.8, 136.7, 136.5, 136.3, 135.9, 135.7, 135.6, 135.3, 135.1, 134.7, 134.6, 134.4, 134.0, 133.9, 133.8, 133.8, 133.6, 133.5, 133.4, 133.2, 133.2, 132.3, 132.1, 132.0, 131.9, 131.5, 129.2, 126.4, 126.2, 111.9, 111.8, 96.6, 96.5, 23.3, 16.2; MALDI-TOF ($Ag^+$): *m/z* = 3384.1 ($[M+Ag]^+$).

**1st generation ethynyl polytriphenylene dendrimer (9):**

To a solution of **8** (0.063 g, 0.019 mmol) in THF (3 mL) was added dropwise a solution of TBAF (0.065 g, 0.250 mmol) in THF (2 mL). The reaction was stirred at RT for 1 h and precipitate in MeOH. The product was dissolved again in THF and repeatedly precipitated in MeOH three times, affording light yellow powder. Yield: 0.034 g, 0.016 mmol, 90%. $^1$H NMR (250 MHz, $C_2D_2Cl_4$) δ 8.57 (s, 8H, aromatic *H*1), 7.80 (s, 4H, aromatic *H*2), 7.64 (d, 4H, *J* = 8.7 Hz, aromatic *H*3), 7.57 - 7.44 (n.r., 24H), 7.26-7.09 (n.r., 28H), 6.96 (d, 8H, *J* = 8.3 Hz, aromatic *H*9), 6.87 (d, 8H, *J* = 8.3 Hz, aromatic *H*10), 3.22 (d, 8H, *J* = 8.8 Hz, C≡C*H*); $^{13}$C NMR (75 MHz, $C_2D_2Cl_4$) δ 144.3, 143.4, 141.3, 140.8, 138.7, 138.2, 137.0, 132.4, 131.7, 131.3, 131.1, 130.5, 130.4, 130.1, 129.9, 129.5, 129.4, 129.2, 128.9, 128.7, 128.5, 128.4, 128.3, 127.5, 127.1, 127.0, 126.8, 119.9, 119.6, 83.7, 83.6, 29.6; MALDI-TOF: *m/z* = 2028.4 ($[M]^+$).

EXAMPLE 3

Synthesis of 2nd generation dendrimers

The synthesis of the 2nd generation dendrimers G2, 10 and 11 is shown in Scheme 3 above.

**2nd generation polytriphenylene dendrimer (G2):**

Dendrimer **9** (0.040 g, 0.020 mmol) and compound **7** (0.122 g, 0.320 mmol, ) were dissolved in o-xylene (2 mL) in a microwave tube under Argon and stirred at 170 ˚C for 8 h. After cooling to RT, the reaction mixture was precipitated in MeOH, then further purified by a column chromatography (PE:DCM = 1:1), affording a light yellow powder. Yield: 0.090 g, 0.019 mmol, 94%.
$^1$H NMR (700 MHz, $CD_2Cl_2$) δ 8.40 (d, 16H, , *J* = 7.8 Hz, aromatic *H*1), 8.06 (s, 8H, aromatic *H*2), 7.73 (s, 4H, aromatic *H*3), 7.67 (ss, 16H, aromatic *H*4), 7.52 - 7.31 (n.r., 84H), 7.16 (d, 16H, *J* = 7.2 Hz, aromatic *H*5), 7.10 - 6.94 (n.r., 76H), 6.87 (d, 8H, *J* = 8.2 Hz, aromatic *H*6), 6.61 (d, 4H, *J* = 8.5 Hz, aromatic *H*7), 6.55 (d, 4H, *J*= 8.7 Hz, aromatic *H*8); $^{13}$C NMR (125 MHz, $CD_2Cl_2$) δ 145.0, 144.8, 144.7, 142.5, 142.4, 142.4, 140.6, 140.3, 140.1, 140.0, 139.9, 138.6, 137.4, 137.0, 132.8, 132.5, 132.4, 132.2, 132.0, 131.9, 131.7, 131.5, 131.2, 131.1, 130.8, 130.5, 130.4, 130.2, 130.0, 129.5, 129.4, 129.3, 129.2, 129.1, 128.8, 128.7, 128.7, 127.7, 127.6, 127.5, 127.4, 127.3, 127.0, 126.9, 126.7, 125.9, 125.5, 124.8, 123.5; MALDI-TOF ($Ag^+$): *m/z* = 4966.8 ($[M+Ag]^+$).

**2nd generation T*i*PSethynyl polytriphenylene dendrimer (10):**

Dendrimer **9** (0.034 g, 0.017 mmol) and compound **6** (0.280 g, 0.377 mmol, ) were dissolved in o-xylene (2 mL) in a microwave tube under Argon and stirred at 170 ˚C for 8 h. After cooling to RT, the reaction mixture was precipitated in MeOH, then further purified by a column chromatography (PE:DCM = 3:1), affording a light yellow powder. Yield: 0.113 g, 0.015 mmol, 88%.
$^1$H NMR (700 MHz, $CD_2Cl_2$) δ 8.51 (s, 16H, aromatic *H*1), 8.05 (s, 8H, aromatic *H*2), 7.71 (t, 16H, *J* = 8.1 Hz, aromatic *H*3), 7.59 (dd, 12H, *J*1 = 4.1 Hz, *J*2 = 8.6 Hz, aromatic *H*4), 7.52 - 7.31 (n.r., 72H), 7.16 - 6.98 (n.r., 80H), 6.86 (d, 8H, *J* = 8.2 Hz, aromatic *H*5), 6.60 (d, 4H, *J* = 8.7 Hz, aromatic *H*6), 6.54 (d, 4H, *J* = 8.7 Hz, aromatic *H*7), 1.15 (s, 336H; TiPS); $^{13}$C NMR (62.5 MHz, $CD_2Cl_2$) δ 149.0, 148.7, 146.8, 146.4, 145.1, 145.0, 144.8, 144.7, 142.5, 142.4, 142.4,

140.6, 140.3, 140.1, 140.0, 139.9, 138.6, 137.4, 137.0, 132.8, 132.5, 132.4, 132.2, 132.0, 131.9, 131.7, 131.5, 131.2, 131.1, 130.8, 130.5, 130.4, 130.2, 130.0, 129.5, 129.4, 129.3, 129.2, 129.1, 128.8, 128.7, 128.7, 127.7, 127.6, 127.5, 127.4, 127.3, 127.0, 126.9, 126.7, 125.9, 125.5, 124.8, 123.5, 111.9, 111.8, 96.6, 96.5, 23.2, 16.4; MALDI-TOF ($Ag^+$): *m*/*z* = 7850.3 ($[M+Ag]^+$).

**2$^{nd}$ generation ethynyl polytriphenylene dendrimer (11):**

To a solution of **10** (0.110 g, 0.014 mmol) in THF (3 mL) was added dropwise a solution of TBAF (0.150 g, 0.575 mmol) in THF (2 mL). The reaction was stirred at RT overnight and poured into 50 mL DCM and 50 mL $H_2O$. The organic phase was concentrated and purified by a column chromatography (PE:DCM=2:3), affording a light yellow powder. Yield: 0.070 g, 0.013 mmol, 94%.

$^1$H NMR (250 MHz, $CD_2Cl_2$) δ 8.53 (s, 16H, aromatic *H*1), 8.06 (s, 8H, aromatic *H*2), 7.70 (d, 12H, *J* = 4.1 Hz, aromatic *H*3), 7.59 (d, 8H, *J* = 8. 6 Hz, aromatic *H*4), 7.52 - 7.29 (n.r., 76H), 7.16 - 6.96 (n.r., 84H), 6.84 (d, 8H, *J* = 8.0 Hz, aromatic *H*5), 6.55 (dd, 8H, *J*1 = 8.8 Hz, *J*2 = 16. 8 Hz, aromatic *H*6), 3.22 (d, 16H, *J* = 4.7 Hz, C=C*H*) ; $^{13}$C NMR (62.5MHz, $CD_2Cl_2$) δ 148.2, 146.8, 146.3, 145.0, 144. 3, 143.4, 143.0, 141.3, 140.9, 138.7, 137.8, 137.0, 136.7, 136.5, 135.9, 135.7, 135.6, 135.3, 135.1, 134.7, 134.6, 134.4, 134.0, 133.9, 133.8, 132.7, 132.6, 131.5, 131.4, 131.2, 130.2, 130.0.3, 132.1, 132.0, 131.9, 131.5, 129.9, 129.5, 129.4, 129.2, 128.9, 128.7, 128.5, 128.4, 128.3, 127.5, 127.1, 127.0, 126.8, 119.9, 119.6, 84.1, 83.8, 31.8; MALDI-TOF: *m*/*z* = 5042.5 ([M]+).

EXAMPLE 4

Synthesis of a 3$^{rd}$ generation dendrimer

The synthesis of the exemplary 3$^{rd}$ generation dendrimer G3 is shown in Scheme 3 above.

**3$^{rd}$ generation polytriphenylene dendrimer (G3):**

Dendrimer **11** (0.053 g, 0.010 mmol) and compound **7** (0.250 g, 0.654 mmol, ) were dissolved in *o*-xylene (3 mL) in a microwave tube under Argon and stirred at 170 ˚C for 8 h. After cooling to RT, the reaction mixture was precipitated in MeOH, then further purified by a GPC column using DCM as the eluent, affording a light yellow powder. Yield: 0.098 g, 0.019 mmol, 90%.

$^1$H NMR (700 MHz, $CD_2Cl_2$) δ 8.41 - 8.34 (n.r., 32H), 8.02 (ss, 24H, aromatic *H*1), 7.74 (s, 4H, aromatic *H*2), 7.69 - 7.65 (n.r., 36H), 7.52 - 6.88 (n.r., 404H), 6.67 - 6.58 (n.r., 16H), 6.49 (dd, 8H, *J*1 = 8.7 Hz, *J*2 = 17.0 Hz, aromatic *H*3); $^{13}$C NMR (75 MHz, $CD_2Cl_2$) δ 144.7, 144.6, 142.4, 142.3, 142.1, 140.5, 140.2, 140.0, 139.9, 138.5, 137.0, 136.9, 132.9, 132.8, 132.3, 132.1, 131.9, 131.8, 131.6, 131.5, 131.4, 131.2, 131.1, 131.04, 130.8, 130.8, 130.7, 130.6, 130.6, 130.5, 130.4, 130.3, 130.1, 129.9, 129.6, 129.4, 129.2, 129.1, 129.0, 128.9, 128.7, 128.6, 128.5, 128.3, 128.3, 128.2, 128.1, 128.0, 127.9, 127.9, 127.9, 127.8, 127.5, 127.4, 127.4, 127.3, 127.2, 127.1, 126.9, 126.9, 126.8, 126.7, 126.7, 126.6, 126.5, 126.5, 125.8, 125.6, 125.5, 124.7, 124.6, 123.6, 123.5; MALDI-TOF ($Ag^+$): *m*/*z* = 11009.8 ($[M+Ag]^+$).

EXAMPLE 5

Preparation of a reference dendrimer

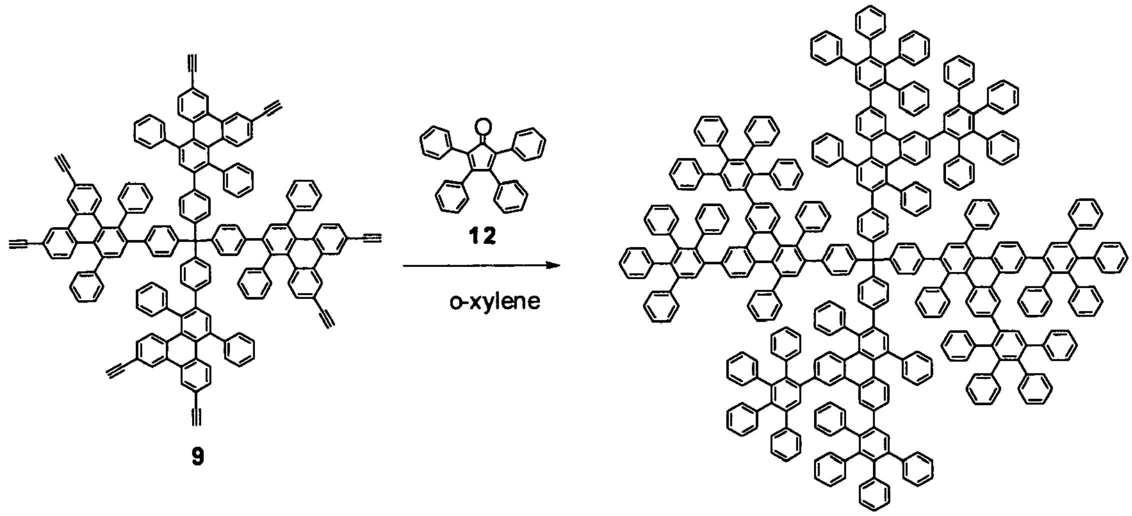


9

G2'

**Scheme 4**

To investigate the influence of the peripheral shielding upon the optical properties of the dendrimers, model compound 1,2,4-triphenyltriphenylene **TTP** was prepared analogous to the first reaction in Scheme 1 from tetraphenylcyclopentadienon (compound 12) and an ethynyl-functionalized phenyl ring. Another polyphenylene derivative, dendrimer **G2'** with a pentaphenyl shell at its periphery was also prepared according to Scheme 4 via analogous Diels-Alder reactions using tetraphenylcyclopentadienon instead of compound (2) in the second step of the preparation.

Dendrimer **G2'** is an end capped version of the first generation dendrimer **G1**, which, however, due to the TTP end caps resembles almost a second generation dendrimer, only lacking the connection at the outmost phenyl rings. Therefore it consists of longer segments than the uncapped first generation species, but is supposed to be more twisted than the second generation dendrimer. Thus the end capped dendrimer **G2**' can be understood as a "*link*" between first and second generation.

EXAMPLE 6

Preparation of a LED device comprising

a polytriphenylene dendrimer of the invention

OLED devices were built in a sandwich geometry (glass/ITO/PEDOT:PSS/dendrimer/TPBi/CsF/Al). The ITO covered glass substrates for the OLEDs were thoroughly cleaned in a variety of organic solvents and exposed to an oxygen plasma dry cleaning step. PEDOT: PSS (Baytron P from Bayer Inc.) layers were spin-coated under ambient conditions and dried according to specifications by Bayer Inc. in argon atmosphere and vacuum. The emissive dendrimer films were spin-cast from a 10 g/l toluene (or chlorobenzene) solution and dried at 80 ˚C for 2 hours in high vacuum conditions. Afterwards, a 10 nm layer of 4,4',4''-Tris(N-3-methylphenyl-N-phenyl-amino)triphenylamine (TPBi; LT-E302 from Rubipy Scientific Inc.) as hole blocking/electron transport layer was evaporated onto the emissive film at a pressure of 4 x $10^{-6}$ mbar. Finally, a thin layer (1 nm) of CsF and aluminum electrodes (100 nm) were evaporated on top of the device via physical vapor deposition at a base pressure of below 2 x $10^{-6}$ mbar.

EXAMPLE 7

Characterization of polytriphenylene dendrimers

Dendrimers **G2, G3,** and **G2'** are readily soluble in chloroform, toluene and tetrahydrofuran (THF), which facilitates the final characterization and confirmation of their structures and purity by NMR spectroscopy and MALDI-TOF spectrometry. MALDI-TOF mass spectra exhibits single intense signals corresponding to the calculated masses

of the target dendrimers **G1**, **G2,** and **G3,** as shown in Fig. 1.

Single crystals of **G1**, suitable for X-ray structure analysis, were grown from the tetrachloroethane and hexane mixtures at room temperature by slow evaporation. The crystal structure is well resolved; it possesses high symmetry and respects little conformational flexibility (Fig. 2). The molecules arrange in an orthorhombic lattice. Each dendrimer has four twisted triphenylene units. The intermolecular distance between two dendrimers is about 3.209 Å. In contrast to most other triphenylene derivatives the inter-dendrimer chromophore-chromophore quenching is thus much reduced.

The UV-vis absorption and photoluminescence properties of all dendrimers were investigated in chloroform solutions ($10^{-3}$ g/l) and solid states. PL excitation spectra were measured using a Shimadzu RF-5301 PC spectrofluorometer. Optical absorption measurements were carried out using a Perkin-Elmer Lambda 9 spectrophotometer. Photoluminescence quantum yield (PLQY) was measured from $10^{-3}$ g/l chloroform solutions by a relative method using quinine sulfate in sulfuric acid solution as a standard. The acquired data are summarized in Table 1.

As shown in Figure 3a, the spectra of the 1,2,4-triphenyltriphenylene (**TTP**) molecule and the first generation dendrimer (**G1**) are almost alike in shape and emission and absorption maxima, which is attributed to the fact that the absorbing and light emitting chromophore units are identical in both cases (**TTPs**). Dendrimers **G2** and **G3,** on the other hand, are increasingly red-shifted in emission and absorption as a consequence of the increasing number of neighboring TTP units on each branch of the molecule. This does lead to an increased $\Pi$-orbital overlap of TTP units of different generations and therefore increases the effective conjugation in the entire molecule. **G2**', which is an end capped version of the first generation dendrimer has its absorption and emission spectra situated well between that of **G1** and **G2.** In fact, a closer look at the chemical structures reveals that **G2'** resemble almost a second generation dendrimer, only lacking the connection at the outmost phenyl rings. For the investigation of possible aggregations PL measurements of different toluene dilutions of all five materials were performed. Over 3 orders of magnitude ($10^{-4}$, $10^{-3}$, and $10^{-2}$ g/l) no concentration dependent effects are observable in any spectra, what is an argument against the formation of excimers or emission artifacts from aggregations. Apparently, the chromophores of different dendrimers are not interacting. Photoluminescence quantum yield measurements (PLQY) in solution reveal an increasing yield scaling with the size of molecules (Tab. 1). This indicates that central chromophores in the larger molecules are more effectively shielded against quenchers than the monomer or in a lower dendrimer generation, where more units are accessible to dynamic quenching.

Figure 3b shows absorption and PL emission spectra of thin films spun from 1 g/L toluene solution leading to films with a thickness of typically 15 nm. Like in solutions, **TTP** and **G1** appear very similar with only a bathochromic shift of 1 to 6 nm compared to solution spectra. Dendrimers **G2** and **G3,** however, display a more pronounced shift of 17 to 18 nm as compared to solution spectra. In addition, dendrimer **G3** also exhibits a stronger pronounced long wavelength tail than the other investigated samples of the 1$^{st}$ and 2$^{nd}$ generation. This leads to the conclusion that solid state packing can lead to increased coupling of individual TTP units from different generations, due to reduced inter TTP units twisting leading to an increased effective conjugation and a more pronounced spectral relaxation behavior in the solid state. Like observed before, the PL spectrum of the **G2'** is located right between **G1** and **G2** with a peak shift of approximately 10 nm.

Table 1 shows a direct comparison of absorption and PL peak positions in solution and thin film. As mentioned in PL, the peakmaxima display an increasing red shift with larger size of the molecules, both in solution and thin film. This bathochromic shift scales with the size of the molecules, which is most likely caused by an increased ability of larger molecules to flatten out in thin films, thus increasing the effective conjugation. It is worth mentioning that **G2** displays a larger red shift compared to **G2'.** This means that the end caps of dentrimer **G1** seem to stay more twisted in films than the additional triphenylene units on dendrimer **G2.** Also remarkable is the huge stokes shift of more than 100 nm for all samples, which is indicative for a rather large difference in the ground- and excited state configuration. The large stokes shift in all samples is caused by the fact that absorption happens mainly from $S_0 \rightarrow S_4$ and emission from $S_1 \rightarrow S_0$.

Amongst the investigated dendrimers **G2** and **G2'** exhibit the best thermal stability as well as good and acceptable, respectively, PLQYs and therefore these two materials were in details tested for their electroluminescent (EL) properties in different device geometries despite the fact that all different generations of dendrimers in principle exhibit EL emission. Since first tests showed certain instabilities at the cathode interface, an additional electron transport/hole blocking layer was applied and the organic light-emitting diodes (OLEDs) and the following geometry was used: ITO/PEDOT:PSS/Dendrimer/TPBi/CsF/Al.

As depicted in Figure 4, OLEDs from **G2** show a favorable blue electroluminescence spectrum with a maximum at 430 nm and a shape similar to photoluminescence. Luminance values of 300 cd/m$^2$ are found at a bias voltage of 8 V and color coordinates of the emission are in a readily visible blue region with CIE 1931 value of (0.19, 0.18). The device displayed an onset of electroluminescence at approximately 6 V and maximum efficiencies of 0.4 cd/A. Figure S2 displays the characteristics of a **G2'** device. The normalized EL spectrum resembles the thin film PL spectrum very well and is located in a deeper blue region than TP-G2 with a maximum at 415 nm. At 10 V driving voltage, a maximum luminance of 400 cd/m$^2$ can be found with an efficiency of 0.1 cd/A. Compared to **G2** this lower efficiency is due to the fact that a remarkable part of the EL spectrum is in the UV region and does not contribute to the luminance value. The

corresponding CIE1931 coordinate of **G2'** is (0.17, 0.10). Like for **G2,** applying higher voltages above 10 V does not lead to higher luminance, but starts degradation of the material. In terms of stability, however, these devices are not yet optimized. Although the hole blocking/electron transport layer already led to increased stability, an additional electron transporter could be incorporated into the active layer in order to get a more balanced carrier injection and thus lower onsets, higher efficiencies and an improved overall performance.

EL spectra (intensity per wavelength interval) were recorded using an ORIEL Multispec spectrometer with an attached ANDOR DB401-UV CCD camera. The current / luminance / voltage (ILV) characteristics were recorded in a customized setup using a Keithley 236 source measure unit for recording the current / voltage characteristics while recording the luminance using a calibrated photodiode attached to an integrating Ulbrich sphere.

Table 1. Optical Data for Dendrimers and Model Compounds

| Sample # | $\Lambda_{max}^{Abs}$ [nm] | | $\Lambda_{max}^{PL}$ [nm] | | Quantum yield [%][a] |
|---|---|---|---|---|---|
| | Solution | Thin film | Solution | Thin film | |
| **TTP** | 289 | 298 | 401 | 402 | 3.8 |
| **G1** | 283 | 293 | 400 | 406 | 6.6 |
| **G2** | 304 | 312 | 414 | 431 | 27.0 |
| **G3** | 299 | 313 | 424 | 442 | 35.2 |
| **G2'** | 312 | 317 | 408 | 418 | 14.8 |
| [a] relative to quinine sulfate dihydrate | | | | | |

PLQY measurements in solution reveal an increasing yield scaling with the size of molecules. This indicates that central chromophores in the larger molecules are more effectively shielded against quenchers than the monomer or small dendrimers, where more units are accessible to dynamic quenching.

## Claims

1. A polytriphenylene dendrimer comprising a core unit with linked diphenyltriphenylene repeating units of the following formula (1)

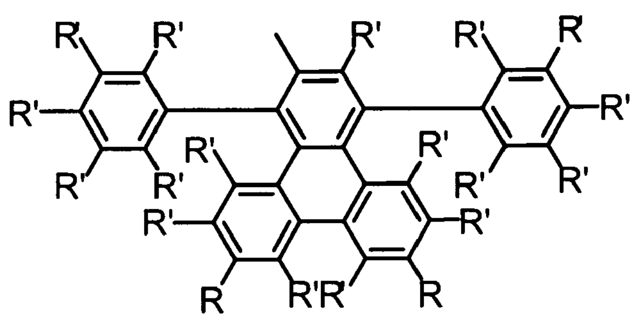

wherein each R' independently is H or a substituent which does not participate in a Diels-Alder reaction, each R independently is H, a substituent which does not participate in a Diels-Alder reaction, an ethynyl group or protected ethynyl group, or a diphenyltriphenylene unit of said formula (1).

2. The polytriphenylene dendrimer according to claim 1, wherein each R' independently is H or a substitent selected from the group consisting of aryl, substituted aryl, heteroaryl, substituted heteroaryl, alkyl, alkylaryl, amino, halo, OH, carboxy, alkoxy, ester, and each R independently is H, a substituent selected from the group consisting of aryl, substituted aryl, heteroaryl, substituted heteroaryl, alkyl, alkylaryl, amino, halo, OH, carboxy, alkoxy, ester, an ethynyl group or protected ethynyl group, or a diphenyltriphenylene unit of said formula (1).

**3.** The polytriphenylene dendrimer according to claim 1 or 2, comprising one of the following structures

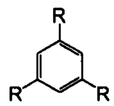 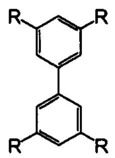 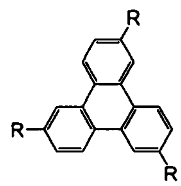 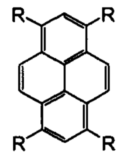 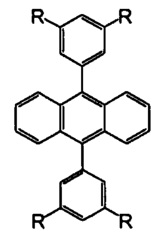 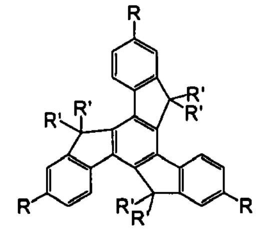

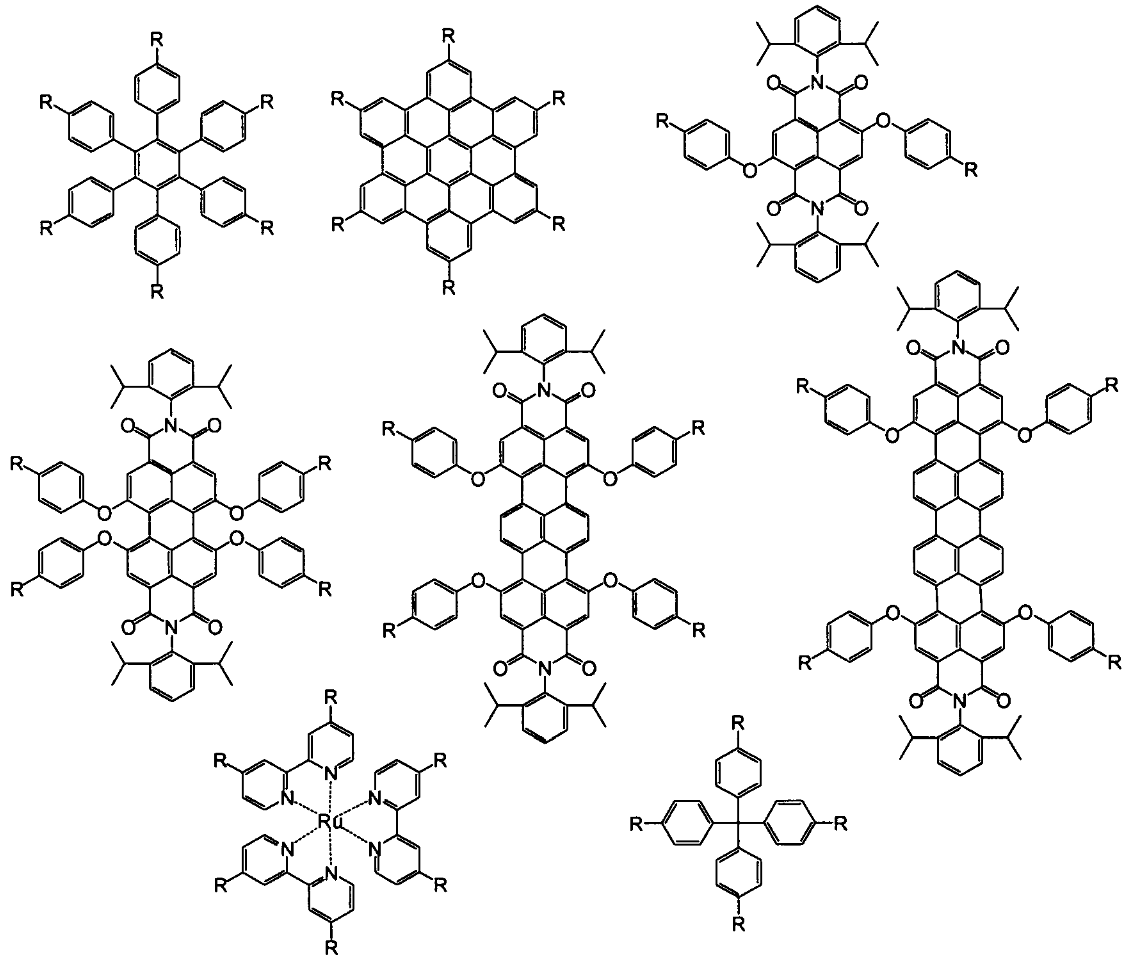

wherein each substituent R of the core unit independently is a polytriphenylene dendron consisting of one or more diphenyltriphenylene units of formula (1) in claim 1 and each R', if present, independently is H or a substituent which does not participate in a Diels-Alder reaction.

**4.** The polytriphenylene dendrimer according to claim 3, comprising a compound of the following formulae (G01) or (G02)

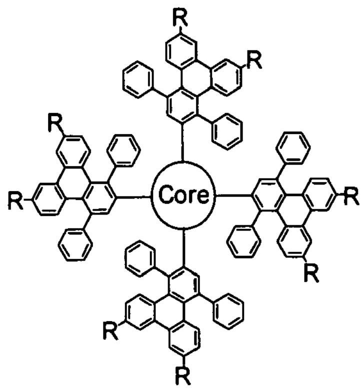

(G01)

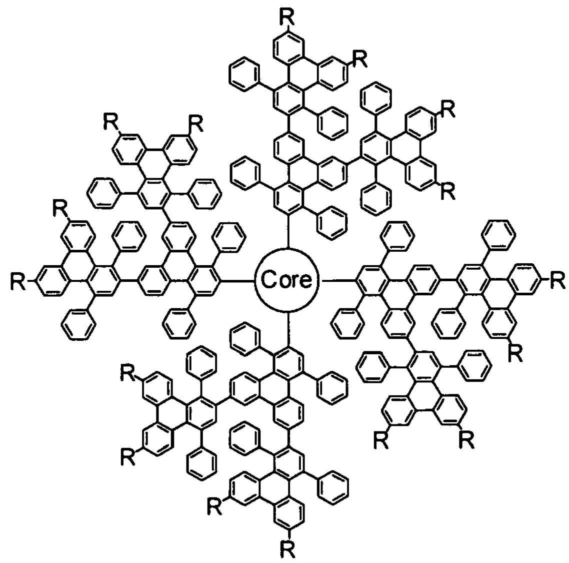

(G02)

wherein all substituents R are the same and as defined in claim 1 or 2.

**5.** The polytriphenylene dendrimer according to claim 4 having the formula

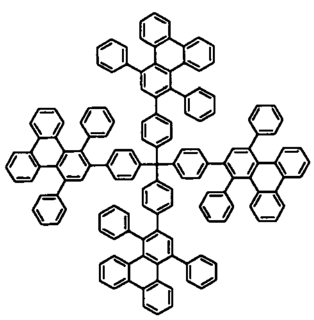

G1

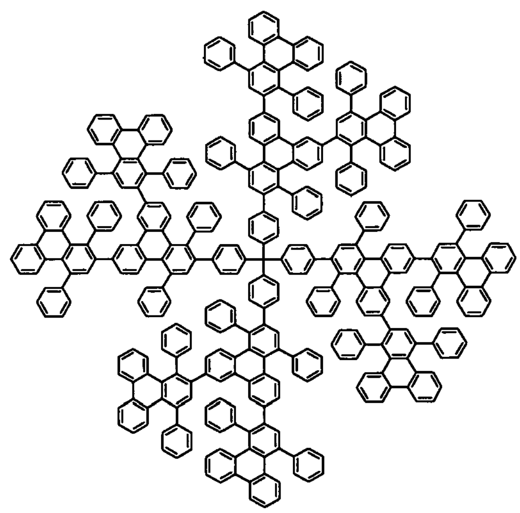

G2

or

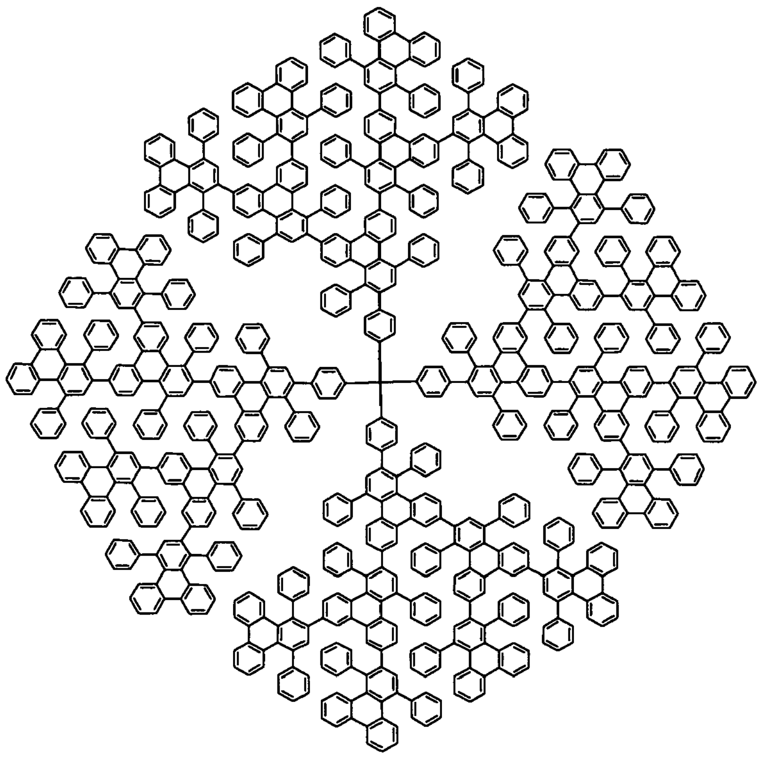

G3 .

6. A polytriphenylene dendrimer acccording to any one of claims 1-5, capable of blue light electroluminescence emission.

7. A method for synthesisizing a polytriphenylene dendrimer comprising:

reacting a compound comprising at least 2, 3 or 4
reactive ethynylphenyl groups and a 1,3-diphenylcyclopentaphenanthrenone of the formula (2)

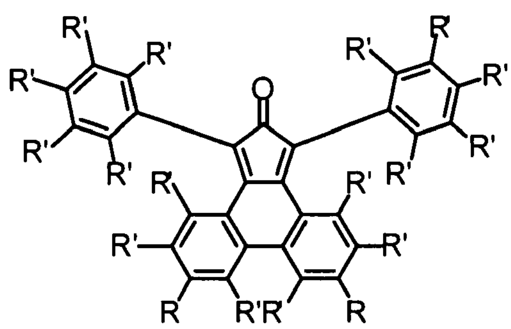

(2)

wherein each R' independently is H or a substituent which does not participate in a Diels-Alder reaction, and each R independently is H, a substituent which does not participate in a Diels-Alder reaction, or a protected ethynyl group,

in a Diels-Alder cycloaddition to form a polytriphenylene dendrimer.

**8.** The method according to claim 7, wherein both R in formula (2) are the same and the resulting polytriphenylene dendrimer has one of the structures of claim 3.

**9.** The method according to claim 7 or 8, wherein both R in formula (2) are a protected ethynyl group and the Diels-Alder cycloaddition results in a first generation polytriphenylene dendrimer comprising protected ethynyl groups R, and which method further comprises

a) deprotecting the ethynyl groups and
b) reacting the deprotected ethynyl groups with the 1,3-diphenylcyclopentaphenanthrenone of the formula (2) as defined in claim 7 or 8 in a Diels-Alder cycloaddition to form a second generation polytriphenylene dendrimer and,
c) if the second generation polytriphenylene dendrimer comprises protected ethynyl groups R due to the presence of such groups in the diphenylcyclopentaphenanthrenone of the above formula (2) used in step b), optionally repeating steps a) and b) once or several times to form polytriphenylene dendrimers of a third or higher generation.

**10.** The method according to claim 9, wherein the substituents R of the diphenylcyclopentaphenanthrenone of formula (2) used in step b) and/or step c) are the same.

**11.** The method according to any one of claims 8-10 comprising:

reacting tetrakis(4-ethynylphenyl)methane and a 1,3-diphenylcyclopentaphenanthrenone of the formula (201)

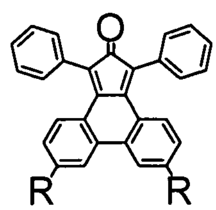

wherein all substituents R are the same and selected from H, an ethynyl group or protected ethynyl group,
in a Diels-Alder cycladdition to form a polytriphenylene dendrimer of the following formula (G01)

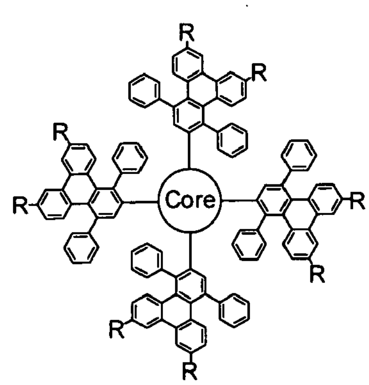

wherein all substituents R are the same and selected from H, an ethynyl group or protected ethynyl group.

**12.** The method according to claim 11, further comprising

a) reacting the polytriphenylene dendrimer of formula (G01)

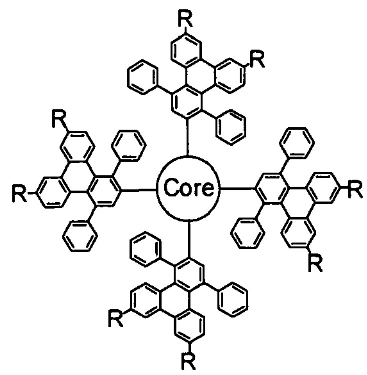

wherein each R is a protected ethynyl group,
with a deprotecting agent to form a polytriphenylene dendrimer of formula (G01)

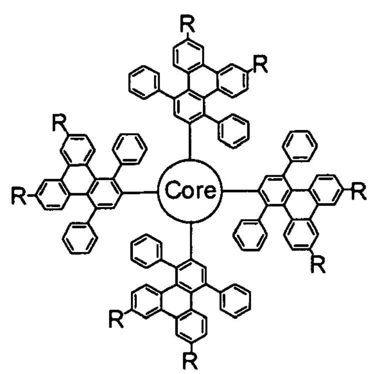

wherein R is an ethynyl group, and
b) further reacting the dendrimer obtained in step a) with a 1,3-diphenylcyclopentaphenanthrenone of the formula (201)

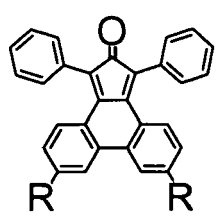

wherein R may be H or a protected ethynyl group,
to form a second generation polytriphenylene dendrimer, and
c) if the second generation polytriphenylene dendrimer comprises protected ethynyl groups R due to the presence of such groups in the diphenylcyclopentaphenanthrenone of the above formula (201), optionally repeating steps a) and b) once or several times to form polytriphenylene dendrimers of a third or higher generation.

**13.** A method for synthesizing the diphenylcyclopentaphenanthrenone of formula (2) as defined in claim 7

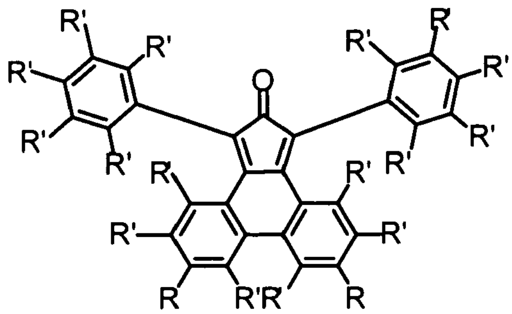

(2)

comprising the steps:

a) brominating an unsubstituted or substituted phenanthrene-9,10-dione to give the corresponding 3,6-dibromophenanthrene-9,10-dione;
b) protecting the dione group with a protecting agent, e.g. dimethylsulfate, to give a protected derivative, e.g. 3,6-dibromo-9,10-dimethoxyphenanthrene;
c) reacting the product of step b), e.g. 3,6-dibromo-9,10-dimethoxyphenanthrene, with a compound providing a protected ethynyl group, e.g. ethynyltriisopropylsilane or ethynyltrimethylsilane, to give the corresponding ethynyl-functionalized product, e.g. 9,10-dimethoxy-3,6-bis-(triisopropylsilyl)ethynyl)phenanthrene or 9,10-dimethoxy-3,6-bis(trimethylsilyl)ethynyl)phenanthrene;
d) removing the methoxy groups or other protecting groups of step b) by reacting with an oxidating agent to give a 3,6-bis(triisopropylsilyl)ethynyl)phenanthrene-9,10-dione or an analogon with another ethynyl-protecting group;
e) reacting the product of step d) with 1,3-diphenylacetone under Knoevenagel condensation to give the diphenylcyclopentaphenanthrenone of the above formula (2) wherein each R is a protected ethynyl group, e.g. a triisopropylsilylethynyl or trimethylsilylethynyl group, and
f) optionally removing the protecting groups, e.g. triisopropylsilyl groups, to give the diphenylcyclopentaphenanthrenone of the above formula (2) wherein each R is an ethynyl group.

**14.** An emissive layer comprising the polytriphenylene dendrimer according to any one of claims 1-6.

**15.** Use of the polytriphenylene dendrimer according to any one of claims 1-6 or of the emissive layer of claim 14 in opto-electronic devices such as organic LED devices, organic photodetectors, organic phototransistors, FET and photovoltaic cells.

**16.** An opto-electronic device, in particular an organic LED device, an organic photodetector, organic phototransistor, FET or a photovoltaic cell, comprising a polytriphenylene dendrimer according to any one of claims 1-6 or an emissive layer according to claim 14.

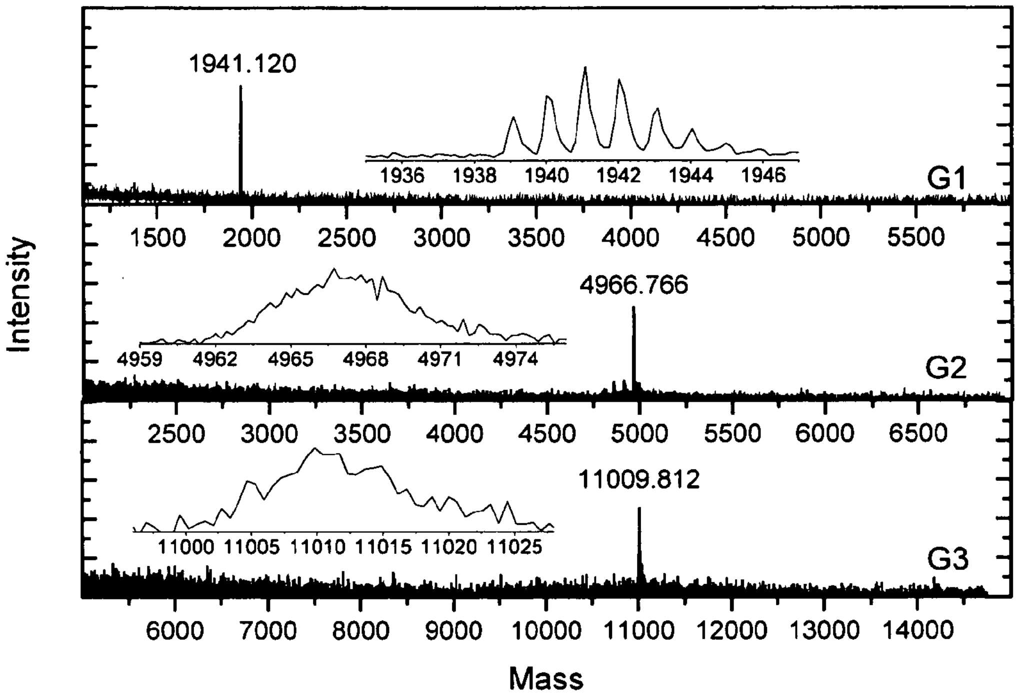
1941.120
1936 1938 1940 1942 1944 1946
G1
1500 2000 2500 3000 3500 4000 4500 5000 5500
4966.766
4959 4962 4965 4968 4971 4974
G2
2500 3000 3500 4000 4500 5000 5500 6000 6500
11009.812
11000 11005 11010 11015 11020 11025
G3
6000 7000 8000 9000 10000 11000 12000 13000 14000
Mass
Intensity

Fig. 1

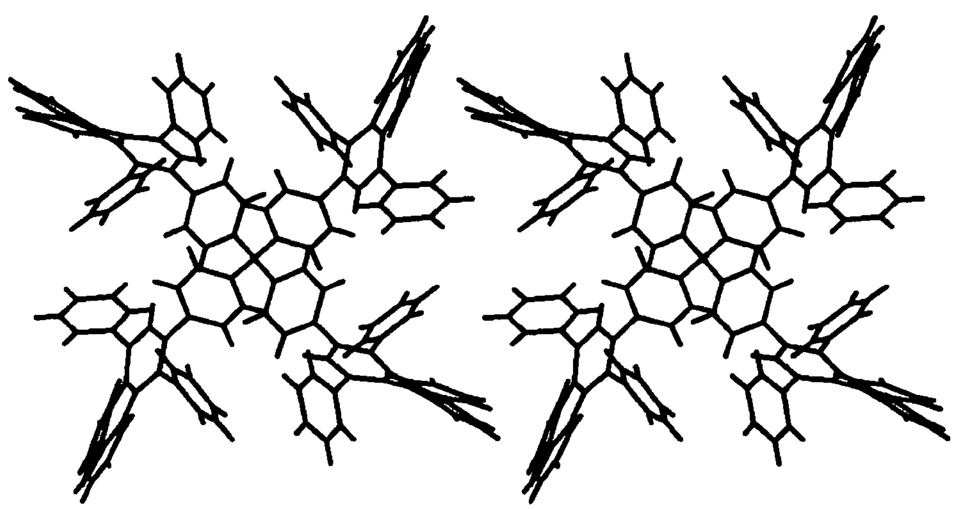

Fig. 2

Fig. 3a

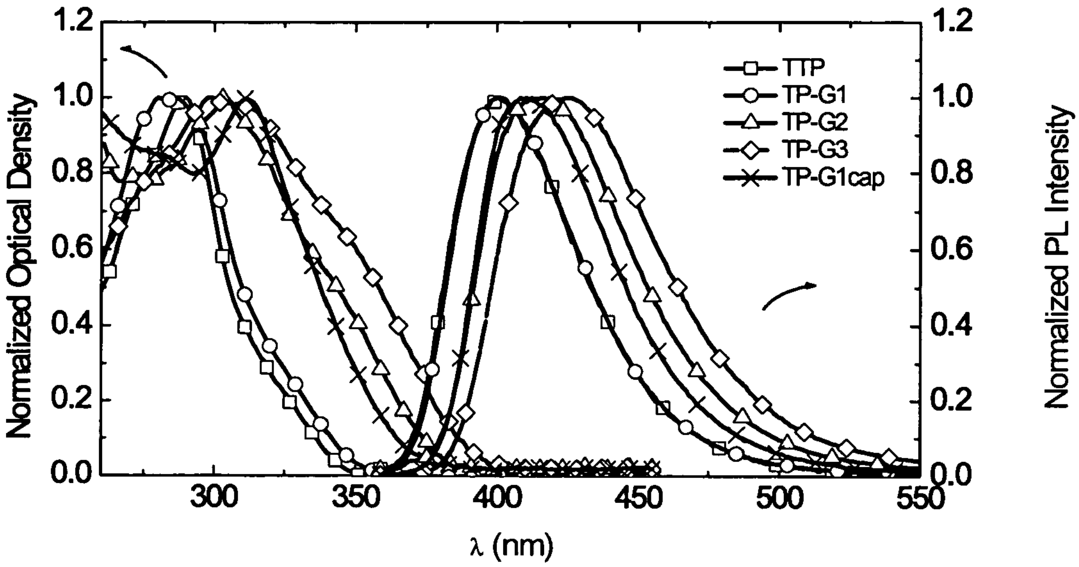
Normalized Optical Density
Normalized PL Intensity
λ (nm)
TTP
TP-G1
TP-G2
TP-G3
TP-G1cap
1.2
1.0
0.8
0.6
0.4
0.2
0.0
300
350
400
450
500
550

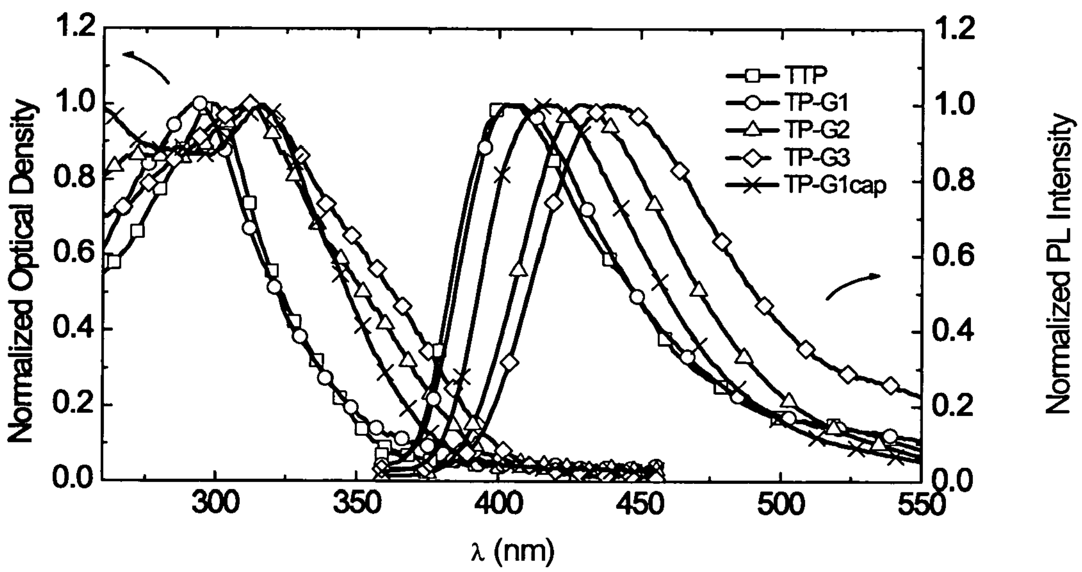
Normalized Optical Density
Normalized PL Intensity
λ (nm)
TTP
TP-G1
TP-G2
TP-G3
TP-G1cap
1.2
1.0
0.8
0.6
0.4
0.2
0.0
300
350
400
450
500
550

Fig. 3b

Fig. 4

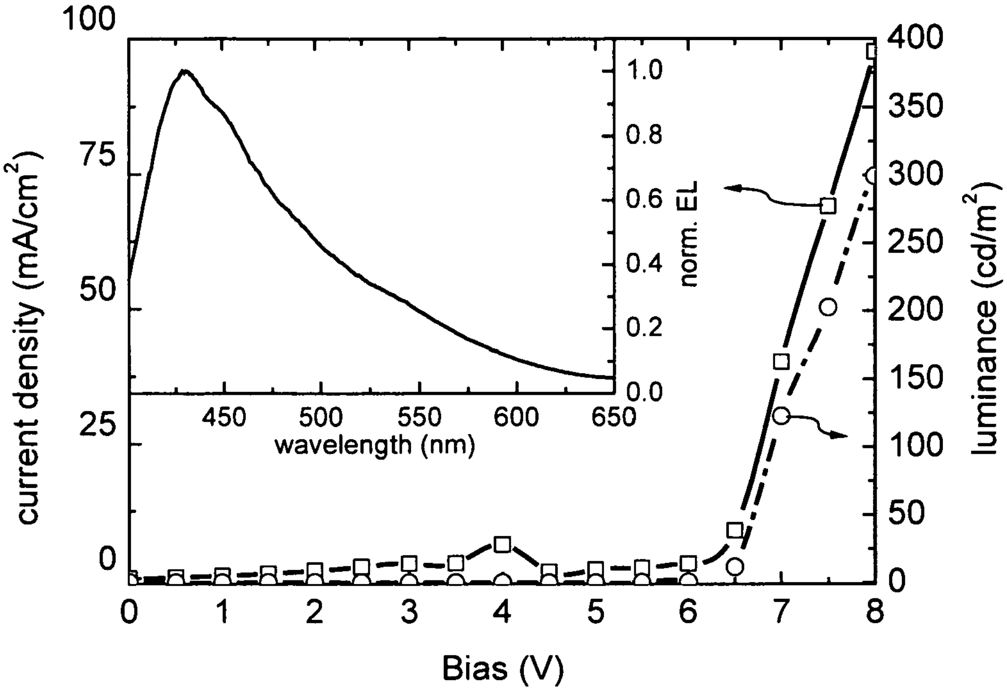
100
75
50
25
0
current density (mA/cm²)
400
350
300
250
200
150
100
50
0
luminance (cd/m²)
0 1 2 3 4 5 6 7 8
Bias (V)
1.0
0.8
0.6
0.4
0.2
0.0
norm. EL
450 500 550 600 650
wavelength (nm)

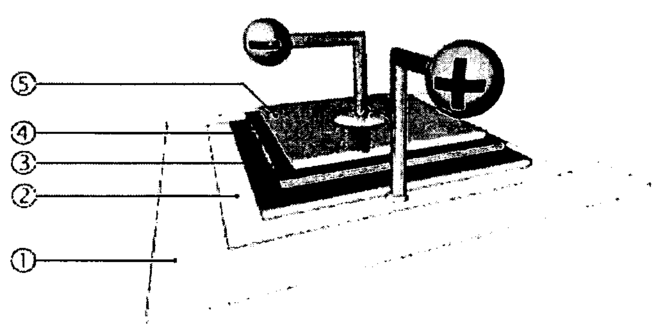
⑤
④
③
②
①

Fig. 5

Europäisches Patentamt
European Patent Office
Office européen des brevets

# PARTIAL EUROPEAN SEARCH REPORT

which under Rule 63 of the European Patent Convention shall be considered, for the purposes of subsequent proceedings, as the European search report

Application Number
EP 08 00 8782

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VON WALTER R ET AL: "Über die Synthese von Mono-, Bis- und Tris-äthinyl-Aromaten und ihre Reaktionen mit Cyclopentadienonen" JUSTUS LIEBIGS ANNALEN DER CHEMIE, VERLAG CHEMIE GMBH, WEINHEIM,; DE, vol. 739, 1 January 1970 (1970-01-01), pages 159-165, XP008097448 ISSN: 0075-4617 * the whole document * ----- | 1-5,7-12 | INV. C09K11/06 H05B33/14 C07C49/755 C08G61/10 H01L51/00 |
| X | US 2005/048318 A1 (SUZUKI KOICHI [JP] ET AL) 3 March 2005 (2005-03-03) * page 9; compound 21 * * page 15; compound 47 * * page 18; compound 56 * * claims 2,3; compounds (VI), (IX), (VII) * ----- | 1-12, 14-16 | |

-/--

TECHNICAL FIELDS SEARCHED (IPC)

C09K
H05B
C07C
C08G
H01L

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 2 December 2009 | Vanier, Cécile |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

Europäisches Patentamt
European Patent Office
Office européen des brevets

# PARTIAL EUROPEAN SEARCH REPORT

Application Number
EP 08 00 8782

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X | ROLAND E BAUER ET AL: "Functionalised Polyphenylene Dendrimers and Their Applications" TOPICS IN CURRENT CHEMISTRY, SPRINGER, BERLIN, DE, vol. 245, 1 January 2005 (2005-01-01), pages 253-286, XP008097400 ISSN: 0340-1022 * pages 256,269 * ----- | 1-12 | |
| X | L'ESPERANCE R P, VAN VEGEN D, DAYAL R, PASCAL R A JR: "A study of Substituent Effects on Hydrogen-to-Arene Nonbonded Interactions" JOURNAL OF ORGANIC CHEMISTRY, vol. 56, 1991, pages 688-694, XP002499535 * the whole document * ----- | 7-12 | TECHNICAL FIELDS SEARCHED (IPC) |
| A | WO 2006/130598 A (UNIVERSAL DISPLAY CORP [US]; KWONG RAYMOND [US]; ALLEYNE BERT [US]) 7 December 2006 (2006-12-07) * paragraphs [0015], [0020] * * claims 1,7,12,15,20,24,37,39 * ----- | 1-12, 14-16 | |
| X | HORNIG F S, KORTH H-G, RAUEN U, DE GROOT H, SUSTMAN R: "synthesis and properties of pH-insensitive fluorescent nitric oxide cheletropic trap (FNOCT)" HELVETICA CHIMICA ACTA, vol. 89, 2006, pages 2281-2296, XP002558081 * page 2284 * ----- | 13 | |

4

EPO FORM 1503 03.82 (P04C10)

Europäisches Patentamt
European Patent Office
Office européen des brevets

**INCOMPLETE SEARCH SHEET C**

Application Number
EP 08 00 8782

Claim(s) completely searchable:
4-5, 11-12

Claim(s) searched incompletely:
1-3, 6-10, 14-16

Reason for the limitation of the search (non-patentable invention(s)):

The present claims 1-3 and 6 relate to an extremely large number of possible compounds. Support and disclosure in the sense of Article 84 and 83 EPC is to be found however for only a very small proportion of the compounds claimed, see compounds G1, G2 page 7 and G3 page 8 and examples pages 19-23. The same reasoning applies, mutatis mutandis, to the subject-matter of product claims 14 and 16 and use claim 15.

In addition, the starting products of the method claimed in claims 7-10 are compounds comprising several ethylphenyl groups reacting with diphenylcyclopentaphenanthrenone derivatives. The starting compounds are not clearly defined because their chemical formulae relate to an extremely large number of possible compounds, while support and disclosure in the sense of Article 83 and 84 EPC have only been provided for a very small proportion of said compunds (see above reasoning). This would require an equally unquantifiable and thus unreasonable amount of experimentation, imposing a severe and undue burden on all those wishing to ascertain the scope of the claim, which is not in compliance with the clarity requirement of Article 84 EPC.

The non-compliance with the substantive provisions is to such an extent, that a meaningful search of the whole claimed subject-matter of the claim could not be carried out (Rule 63 EPC and Guidelines B-VIII, 3). The extent of the search was consequently limited.

The search of claims 1-3, 6, 14-16 was restricted to those claimed compounds which appear to be supported and a generalisation of their structural formulae, namely dendrimers comprising (di)phenyltriphenylene repeating units linked to an aromatic core. THe search of method claims 7-10 was limited to the corresponding starting materials.

-----

Further limitation of the search

Claim(s) completely searchable:
4-5, 11-12

Claim(s) searched incompletely:
1-3, 6-10, 14-16

Reason for the limitation of the search:

The present claims 1-3 and 6 relate to an extremely large number of possible compounds. Support and disclosure in the sense of Article 84 and 83 EPC is to be found however for only a very small proportion of the compounds claimed, see compounds G1, G2 page 7 and G3 page 8 and examples

Europäisches Patentamt
European Patent Office
Office européen des brevets

**INCOMPLETE SEARCH SHEET C**

Application Number
EP 08 00 8782

pages 19-23. The same reasoning applies, mutatis mutandis, to the subject-matter of product claims 14 and 16 and use claim 15.

In addition, the starting products of the method claimed in claims 7-10 are compounds comprising several ethylphenyl groups reacting with diphenylcyclopentaphenanthrenone derivatives. The starting compounds are not clearly defined because their chemical formulae relate to an extremely large number of possible compounds, while support and disclosure in the sense of Article 83 and 84 EPC have only been provided for a very small proportion of said compunds (see above reasoning). This would require an equally unquantifiable and thus unreasonable amount of experimentation, imposing a severe and undue burden on all those wishing to ascertain the scope of the claim, which is not in compliance with the clarity requirement of Article 84 EPC.

The non-compliance with the substantive provisions is to such an extent, that a meaningful search of the whole claimed subject-matter of the claim could not be carried out (Rule 63 EPC and Guidelines B-VIII, 3). The extent of the search was consequently limited.

The search of claims 1-3, 6, 14-16 was restricted to those claimed compounds which appear to be supported and a generalisation of their structural formulae, namely dendrimers comprising (di)phenyltriphenylene repeating units linked to an aromatic core. The search of method claims 7-10 was limited to the corresponding starting materials.

Europäisches Patentamt
European Patent Office
Office européen des brevets

Application Number
EP 08 00 8782

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 08 00 8782

The Search Division considers that the present European patent application does not comply with the requirements of unity of invention and relates to several inventions or groups of inventions, namely:

1. claims: 1-12, 14-16

   Blue emitting polytriphenylene dendrimers, their synthesis, their use in electroluminescent devices and said devices.

   ---

2. claim: 13

   Synthesis of a diphenylcyclopentaphenanthrenone having 2 protective ethynyl groups in 3 and 6 positions.

   ---

## ANNEX TO THE EUROPEAN SEARCH REPORT ON EUROPEAN PATENT APPLICATION NO. EP 08 00 8782

This annex lists the patent family members relating to the patent documents cited in the above-mentioned European search report.
The members are as contained in the European Patent Office EDP file on
The European Patent Office is in no way liable for these particulars which are merely given for the purpose of information.

02-12-2009

| Patent document cited in search report | Publication date | Patent family member(s) | Publication date |
|---|---|---|---|
| US 2005048318 A1 | 03-03-2005 | NONE | |
| WO 2006130598 A | 07-12-2006 | CN 101203583 A | 18-06-2008 |
| | | EP 1888708 A2 | 20-02-2008 |
| | | JP 2008543086 T | 27-11-2008 |
| | | KR 20080013934 A | 13-02-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006130598 A
- WO 2008012250 A


### Non-patent literature cited in the description


- **Freudenmann et al.** *J. Mat. Chem.,* 2001, vol. 11, 1618